(19) Europäisches Patentamt
European Patent Office
Office européen des brevets

(11) EP 3 971 294 A1

(12) EUROPEAN PATENT APPLICATION

(43) Date of publication:
**23.03.2022 Bulletin 2022/12**

(21) Application number: **20306043.9**

(22) Date of filing: **17.09.2020**

(51) International Patent Classification (IPC):
***C12N 15/62*** *(2006.01)* ***C12Q 1/70*** *(2006.01)*

(52) Cooperative Patent Classification (CPC):
(C-Sets available)
**C07K 14/4702; C12N 5/0602; C12N 15/62;**
C12Q 1/70; C12Q 1/703; C12Q 2600/136 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **UNIVERSITE DE MONTPELLIER
34090 Montpellier (FR)**
- **Centre national de la recherche scientifique
75016 Paris (FR)**

(72) Inventors:
- **NISOLE, Sébastien
34170 Castelnau-Le-Lez (FR)**
- **ARHEL, Nathalie
34170 Castelanau-Le-Lez (FR)**
- **FERNANDEZ, Juliette
34090 Montpellier (FR)**

(74) Representative: **Cabinet Becker et Associés
25, rue Louis le Grand
75002 Paris (FR)**

# (54) PROCESSES FOR MONITORING TRAFFICKING EVENTS DURING INFECTION AND INNATE IMMUNE RESPONSE

(57) The present invention relates to processes for monitoring viral infections and innate immune responses. More particularly, the present invention relates to processes for monitoring the translocation of proteins of interest to given subcellular components, wherein the translocated proteins are indicative of infection or sensing.

EP 3 971 294 A1

(52) Cooperative Patent Classification (CPC): (Cont.)

C-Sets
C12Q 1/70, C12Q 2561/107, C12Q 2563/107

SEQUENCE LISTING

<110> UNIVERSITE DE MONTPELLIER

<120> PROCESSES FOR MONITORING TRAFFICKING EVENTS DURING INFECTION AND INNATE IMMUNE RESPONSE

<130> B3366EP00

<160> 56

<170> PatentIn version 3.5

<210> 1
<211> 171
<212> PRT
<213> ARTIFICIAL SEQUENCE

<220>
<223> NanoLuc

<400> 1

```
Met Val Phe Thr Leu Glu Asp Phe Val Gly Asp Trp Glu Gln Thr Ala
1               5                   10                  15

Ala Tyr Asn Leu Asp Gln Val Leu Glu Gln Gly Gly Val Ser Ser Leu
            20                  25                  30

Leu Gln Asn Leu Ala Val Ser Val Thr Pro Ile Gln Arg Ile Val Arg
        35                  40                  45

Ser Gly Glu Asn Ala Leu Lys Ile Asp Ile His Val Ile Ile Pro Tyr
    50                  55                  60

Glu Gly Leu Ser Ala Asp Gln Met Ala Gln Ile Glu Glu Val Phe Lys
65                  70                  75                  80

Val Val Tyr Pro Val Asp Asp His His Phe Lys Val Ile Leu Pro Tyr
                85                  90                  95

Gly Thr Leu Val Ile Asp Gly Val Thr Pro Asn Met Leu Asn Tyr Phe
            100                 105                 110

Gly Arg Pro Tyr Glu Gly Ile Ala Val Phe Asp Gly Lys Lys Ile Thr
        115                 120                 125

Val Thr Gly Thr Leu Trp Asn Gly Asn Lys Ile Ile Asp Glu Arg Leu
    130                 135                 140

Ile Thr Pro Asp Gly Ser Met Leu Phe Arg Val Thr Ile Asn Gly Val
145                 150                 155                 160
```

Thr Gly Trp Arg Leu Cys Glu Arg Ile Leu Ala
165 170

<210> 2
<211> 13
<212> PRT
<213> ARTIFICIAL SEQUENCE

<220>
<223> alpha fragment

<400> 2

Gly Val Thr Gly Trp Arg Leu Cys Glu Arg Ile Leu Ala
1 5 10

<210> 3
<211> 158
<212> PRT
<213> ARTIFICIAL SEQUENCE

<220>
<223> Centauri fragment

<400> 3

Met Val Phe Thr Leu Glu Asp Phe Val Gly Asp Trp Glu Gln Thr Ala
1 5 10 15

Ala Tyr Asn Leu Asp Gln Val Leu Glu Gln Gly Gly Val Ser Ser Leu
20 25 30

Leu Gln Asn Leu Ala Val Ser Val Thr Pro Ile Gln Arg Ile Val Arg
35 40 45

Ser Gly Glu Asn Ala Leu Lys Ile Asp Ile His Val Ile Ile Pro Tyr
50 55 60

Glu Gly Leu Ser Ala Asp Gln Met Ala Gln Ile Glu Glu Val Phe Lys
65 70 75 80

Val Val Tyr Pro Val Asp Asp His His Phe Lys Val Ile Leu Pro Tyr
85 90 95

Gly Thr Leu Val Ile Asp Gly Val Thr Pro Asn Met Leu Asn Tyr Phe
100 105 110

Gly Arg Pro Tyr Glu Gly Ile Ala Val Phe Asp Gly Lys Lys Ile Thr
115 120 125

Val Thr Gly Thr Leu Trp Asn Gly Asn Lys Ile Ile Asp Glu Arg Leu
130 135 140

Ile Thr Pro Asp Gly Ser Met Leu Phe Arg Val Thr Ile Asn
145 150 155

<210> 4
<211> 231
<212> PRT
<213> ARTIFICIAL SEQUENCE

<220>
<223> GFP

<400> 4

Met Val Ser Lys Gly Glu Glu Leu Phe Thr Gly Val Val Pro Ile Leu
1 5 10 15

Val Glu Leu Asp Gly Asp Val Asn Gly His Lys Phe Ser Val Arg Gly
20 25 30

Glu Gly Glu Gly Asp Ala Thr Ile Gly Lys Leu Thr Leu Lys Phe Ile
35 40 45

Cys Thr Thr Gly Lys Leu Pro Val Pro Trp Pro Thr Leu Val Thr Thr
50 55 60

Leu Thr Tyr Gly Val Gln Cys Phe Ser Arg Tyr Pro Asp His Met Lys
65 70 75 80

Arg His Asp Phe Phe Lys Ser Ala Met Pro Glu Gly Tyr Val Gln Glu
85 90 95

Arg Thr Ile Ser Phe Lys Asp Asp Gly Lys Tyr Lys Thr Arg Ala Val
100 105 110

Val Lys Phe Glu Gly Asp Thr Leu Val Asn Arg Ile Glu Leu Lys Gly
115 120 125

Thr Asp Phe Lys Glu Asp Gly Asn Ile Leu Gly His Lys Leu Glu Tyr
130 135 140

Asn Phe Asn Ser His Asn Val Tyr Ile Thr Ala Asn Lys Gln Lys Asn
145 150 155 160

Gly Ile Lys Ala Asn Phe Thr Val Arg His Asn Val Glu Asp Gly Ser
165 170 175

Val Gln Leu Ala Asp His Tyr Gln Gln Asn Thr Pro Ile Gly Asp Gly
180 185 190

Pro Val Leu Leu Pro Asp Asn His Tyr Leu Ser Thr Gln Thr Val Leu
        195                 200                 205

Ser Lys Asp Pro Asn Glu Lys Arg Asp His Met Val Leu His Glu Tyr
    210                 215                 220

Val Asn Ala Ala Gly Ile Thr
225                 230

<210> 5
<211> 16
<212> PRT
<213> ARTIFICIAL SEQUENCE

<220>
<223> alpha fragment

<400> 5

Arg Asp His Met Val Leu His Glu Tyr Val Asn Ala Ala Gly Ile Thr
1               5                   10                  15

<210> 6
<211> 215
<212> PRT
<213> ARTIFICIAL SEQUENCE

<220>
<223> Centauri fragment

<400> 6

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Arg Gly
            20                  25                  30

Glu Gly Glu Gly Asp Ala Thr Ile Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Arg His Asp Phe Phe Lys Ser Ala Met Pro Glu Gly Tyr Val Gln Glu
                85                  90                  95

Arg Thr Ile Ser Phe Lys Asp Asp Gly Lys Tyr Lys Thr Arg Ala Val
            100                 105                 110

Val Lys Phe Glu Gly Asp Thr Leu Val Asn Arg Ile Glu Leu Lys Gly
115 120 125

Thr Asp Phe Lys Glu Asp Gly Asn Ile Leu Gly His Lys Leu Glu Tyr
130 135 140

Asn Phe Asn Ser His Asn Val Tyr Ile Thr Ala Asn Lys Gln Lys Asn
145 150 155 160

Gly Ile Lys Ala Asn Phe Thr Val Arg His Asn Val Glu Asp Gly Ser
165 170 175

Val Gln Leu Ala Asp His Tyr Gln Gln Asn Thr Pro Ile Gly Asp Gly
180 185 190

Pro Val Leu Leu Pro Asp Asn His Tyr Leu Ser Thr Gln Thr Val Leu
195 200 205

Ser Lys Asp Pro Asn Glu Lys
210 215

<210> 7
<211> 19
<212> DNA
<213> ARTIFICIAL SEQUENCE

<220>
<223> forward primer

<400> 7
ccagtactac ggttaaggc 19

<210> 8
<211> 94
<212> DNA
<213> ARTIFICIAL SEQUENCE

<220>
<223> Reverse primer

<400> 8
gaaacataca tatgctatgt aatcccagca gcatttacgt actcatgaag gaccatgtgg 60

tcacgagcgg ccgcatcctc atcctgtcta cttg 94

<210> 9
<211> 94
<212> DNA
<213> ARTIFICIAL SEQUENCE

<220>
<223> Reverse primer

<400> 9
gaaacataca tatgttacgc cagaatgcgt tcgcacagcc gccagccggt cactccgtgg 60

tcacgagcgg ccgcatcctc atcctgtcta cttg 94

<210> 10
<211> 20
<212> DNA
<213> ARTIFICIAL SEQUENCE

<220>
<223> primer

<400> 10
gatcggatcc cgccaccatg 20

<210> 11
<211> 64
<212> DNA
<213> ARTIFICIAL SEQUENCE

<220>
<223> primer

<400> 11
aagagcgtaa tctggaacat cgtatgggta gccggcgcct ttctcgtttg ggtctttgct 60

cagc 64

<210> 12
<211> 20
<212> DNA
<213> ARTIFICIAL SEQUENCE

<220>
<223> MH531

<400> 12
tgtgtgcccg tctgttgtgt 20

<210> 13
<211> 20
<212> DNA
<213> ARTIFICIAL SEQUENCE

<220>
<223> MH532

<400> 13
gagtcctgcg tcgagagagc 20

<210> 14
<211> 20
<212> DNA
<213> ARTIFICIAL SEQUENCE

<220>
<223> LRT-P

<400> 14
cagtggcgcc cgaacaggga 20

<210> 15
<211> 23
<212> DNA
<213> ARTIFICIAL SEQUENCE

<220>
<223> MH535

<400> 15
aactagggaa cccactgctt aag 23

<210> 16
<211> 24
<212> DNA
<213> ARTIFICIAL SEQUENCE

<220>
<223> MH536

<400> 16
tccacagatc aaggatatct tgtc 24

<210> 17
<211> 30
<212> DNA
<213> ARTIFICIAL SEQUENCE

<220>
<223> MH603

<400> 17
acactacttg aagcactcaa ggcaagcttt 30

<210> 18
<211> 20
<212> DNA
<213> ARTIFICIAL SEQUENCE

<220>
<223> ß actine for

<400> 18
aacaccccag ccatgtacgt 20

<210> 19
<211> 24
<212> DNA
<213> ARTIFICIAL SEQUENCE

<220>
<223> ß actine rev

<400> 19
cggtgaggat cttcatgagg tagt 24

<210> 20
<211> 22
<212> DNA
<213> ARTIFICIAL SEQUENCE

<220>
<223> ß actine probe

<400> 20
ccagccaggt ccagacgcag ga 22

<210> 21
<211> 21
<212> DNA
<213> ARTIFICIAL SEQUENCE

<220>
<223> SB704

<400> 21
tgctgggatt acaggcgtga g 21

<210> 22
<211> 41
<212> DNA
<213> ARTIFICIAL SEQUENCE

<220>
<223> L-M667 FDN modified

<400> 22
atgccacgta agcgaaactt tccgctgggg actttccagg g 41

<210> 23
<211> 24
<212> DNA
<213> ARTIFICIAL SEQUENCE

<220>
<223> Alu 1

<400> 23
tcccagctac tggggaggct gagg 24

<210> 24
<211> 25
<212> DNA
<213> ARTIFICIAL SEQUENCE

<220>
<223> Alu 2

<400> 24
gcctcccaaa gtgctgggat tacag 25

<210> 25

<211> 19
<212> DNA
<213> ARTIFICIAL SEQUENCE

<220>
<223> Lamda T

<400> 25
atgccacgta agcgaaact 19

<210> 26
<211> 20
<212> DNA
<213> ARTIFICIAL SEQUENCE

<220>
<223> U5-specific primer

<400> 26
ctgactaaaa gggtctgagg 20

<210> 27
<211> 30
<212> DNA
<213> ARTIFICIAL SEQUENCE

<220>
<223> nested probe

<400> 27
ttaagcctca ataaagcttg ccttgagtgc 30

<210> 28
<211> 25
<212> DNA
<213> ARTIFICIAL SEQUENCE

<220>
<223> pol forward

<400> 28
tttagatgga atagataagg cccaa 25

<210> 29
<211> 26
<212> DNA
<213> ARTIFICIAL SEQUENCE

<220>
<223> pol reverse

<400> 29
cagctggcta actatttctt ttgcta 26

<210> 30
<211> 27
<212> DNA
<213> ARTIFICIAL SEQUENCE

<220>
<223> pol probe

<400> 30
aatcactagc cattgctctc caattac 27

<210> 31
<211> 20
<212> DNA
<213> ARTIFICIAL SEQUENCE

<220>
<223> RPL13A-Fq

<400> 31
aacagctcat gaggctacgg 20

<210> 32
<211> 20
<212> DNA
<213> ARTIFICIAL SEQUENCE

<220>
<223> RPL13A-Rq

<400> 32
tgggtcttga ggacctctgt 20

<210> 33
<211> 20
<212> DNA
<213> ARTIFICIAL SEQUENCE

<220>
<223> Pin1-Fq

<400> 33
gagaagatca cccggaccaa 20

<210> 34
<211> 20
<212> DNA
<213> ARTIFICIAL SEQUENCE

<220>
<223> Pin1-Rq

<400> 34
aaagtcctcc tctcccgact 20

<210> 35
<211> 20
<212> DNA
<213> ARTIFICIAL SEQUENCE

<220>
<223> CypA-Fq

<400> 35
gccgaggaaa accgtgtact 20

<210> 36
<211> 22
<212> DNA
<213> ARTIFICIAL SEQUENCE

<220>
<223> CypA-Rq

<400> 36
gtctgcaaac agctcaaagg ag 22

<210> 37
<211> 20
<212> DNA
<213> ARTIFICIAL SEQUENCE

<220>
<223> TRN-1-Fq

<400> 37
ttcgaatgga tcgcctgctt 20

<210> 38
<211> 20
<212> DNA
<213> ARTIFICIAL SEQUENCE

<220>
<223> TRN-1-Rq

<400> 38
ccgtcctcga tcggtgaaaa 20

<210> 39
<211> 20
<212> DNA
<213> ARTIFICIAL SEQUENCE

<220>
<223> TNPO3-Fq

<400> 39
cagctggaac cagaccatga 20

<210> 40
<211> 20
<212> DNA
<213> ARTIFICIAL SEQUENCE

<220>
<223> TNPO3-Rq

<400> 40
cagctggaac cagaccatga 20

<210> 41
<211> 20
<212> DNA
<213> ARTIFICIAL SEQUENCE

<220>
<223> IPO5-Fq

<400> 41
ttgcgtcctc acttggaagc 20

<210> 42
<211> 20
<212> DNA
<213> ARTIFICIAL SEQUENCE

<220>
<223> IPO5-Rq

<400> 42
aagtccgcaa gccattcgat 20

<210> 43
<211> 20
<212> DNA
<213> ARTIFICIAL SEQUENCE

<220>
<223> IPO7-Fq

<400> 43
cagaggagcg gagtccattg 20

<210> 44
<211> 21
<212> DNA
<213> ARTIFICIAL SEQUENCE

<220>
<223> IPO7-Rq

<400> 44
cccacttctt gcatttccac c 21

<210> 45
<211> 20
<212> DNA
<213> ARTIFICIAL SEQUENCE

<220>
<223> MAP 1A-Fq

<400> 45
cttcccggag gtttgaggac 20

<210> 46

<211> 20
<212> DNA
<213> ARTIFICIAL SEQUENCE

<220>
<223> MAP 1A-Rq

<400> 46
agagaggctt cggtcaggat 20

<210> 47
<211> 20
<212> DNA
<213> ARTIFICIAL SEQUENCE

<220>
<223> MAP 1S-Fq

<400> 47
tcctggagga gctcgaaaga 20

<210> 48
<211> 20
<212> DNA
<213> ARTIFICIAL SEQUENCE

<220>
<223> MAP 1S-Rq

<400> 48
ggagaaggtg gcagagtgtc 20

<210> 49
<211> 21
<212> DNA
<213> ARTIFICIAL SEQUENCE

<220>
<223> CKAP1-Fq

<400> 49
cctcaccatc gctgagttca a 21

<210> 50
<211> 22
<212> DNA
<213> ARTIFICIAL SEQUENCE

<220>
<223> CKAP1-Rq

<400> 50
tccagcttgc tgtagaactt gt 22

<210> 51
<211> 20
<212> DNA
<213> ARTIFICIAL SEQUENCE

<220>
<223> WIRE-Fq

<400> 51
gcgccctctt acaggacatt 20

<210> 52
<211> 20
<212> DNA
<213> ARTIFICIAL SEQUENCE

<220>
<223> WIRE-Rq

<400> 52
cttctcgagg atgggagcac 20

<210> 53
<211> 26
<212> DNA
<213> ARTIFICIAL SEQUENCE

<220>
<223> BP2-Fq

<400> 53
acaatggaat taaagccctt aaatgt 26

<210> 54
<211> 28
<212> DNA
<213> ARTIFICIAL SEQUENCE

<220>
<223> BP2-Rq

<400> 54
gaaacaatca gctacttctt tagtttta 28

<210> 55
<211> 20
<212> DNA
<213> ARTIFICIAL SEQUENCE

<220>
<223> KPNB1-Fq

<400> 55
gtcacaaacc ccaacagcac 20

<210> 56
<211> 20
<212> DNA
<213> ARTIFICIAL SEQUENCE

<220>
<223> KPNB1-Rq

<400> 56
agccactcgt accctcgtat 20

## Description

The present invention relates to processes for monitoring viral infections and innate immune responses. More particularly, the present invention relates to processes for monitoring the translocation of proteins of interest to given subcellular components, wherein the translocated proteins are indicative of infection or sensing. Particularly, the processes of the invention allow to monitor the viral translocation of a given virus to a subcellular component of interest, particularly to the nucleus, and the translocation of transcription factors in the nucleus. These processes are particularly useful for screening antiviral candidate molecules and molecules able to stimulate, modulate or inhibit innate immunity.

## Background of the invention

Protein movement between different subcellular compartments is an essential aspect of biological processes, including transcriptional and metabolic regulation, and immune response. Particularly, protein movement is a critical component of infection. Any intracellular infection, whether viral or bacterial, stimulates innate immunity signaling pathways triggered by the detection of pathogen-associated molecular motifs by endosomal or cytosolic surface receptors. These signaling pathways converge on the nuclear translocation of transcription factors (mainly IRF3 and NF-κB), which then stimulate the expression of type I, type II and type III interferons (hereafter, interferons or IFN) and proinflammatory cytokines. Interferons can then bind to the IFN receptor at the surface of cells and trigger a second wave of signaling pathways that lead to the translocation of transcription factors STAT1/STAT2/IRF9 for IFN alpha, beta et lambda and STAT1/STAT1/IRF9 for IFN gamma.

Several tools exist to measure the activation of innate immune signaling pathways, but these are based on reporter systems that measure transcriptional activity (e.g. ISRE-luciferase) and are not robust for screening molecules because they are particularly sensitive to genotoxic stress inducing agents.

Among infections, those by viruses represent a major burden for public health, and a constant threat to humans. The recent emergence and re-emergence of viruses in the human population has highlighted the need to develop broader panels of therapeutic molecules. The actual therapeutic arsenal to fight viral infections is extremely limited and recent viral epidemics and pandemics have demonstrated the need to develop innovative assays to allow for faster and better high-throughput primary screening aiming at the discovery of novel antiviral molecules. Indeed, viral titration, reverse transcription quantitative PCR (RT-qPCR) or immunostaining and fluorescence imaging have been used extensively to measure viral replication. However, testing hundreds or thousands of conditions with these methods can be challenging. Alternatively, viral replication can be inferred from the measurement of virus cytopathic effects, but this readout is limited to lytic viruses. In addition, this method estimates viral replication only at the latest time point when the lysis of infected cells occurs. The methods used nowadays are therefore not efficient to screen antiviral molecules.

To this extent, the knowledge of the translocation of certain important molecules offers an attractive opportunity for the development of therapeutics. Particularly, the knowledge of the translocation and trafficking of viruses and viral particles in the cell would be of interest to identify antiviral molecules, just as knowledge of the innate immunity signaling pathway would contribute to screen modulators of innate immunity.

Thus, a need exists for processes to quantify the subcellular translocation of viruses and transcription factors for application in molecular screening. These processes would be particularly interesting for the development of new antivirals, and in the treatment of inflammatory diseases and cancers.

## Summary of the invention

The intracellular trafficking of functional proteins or virus plays a key role in regulating gene expressions in response to extracellular signals in eukaryotic cells, particularly to activate the adequate inflammation pathways. The inventors have now developed specific tools allowing to evaluate with accuracy the translocation of virus or proteins of interest from a cellular compartment to another. More particularly, the inventors have developed new protein complementation assays (PCA) to monitor either virus trafficking or innate signaling in infected or stimulated cells. The present invention is based on the reconstitution of split fragments of a protein reporter (e.g., bioluminescent proteins) when a translocation of interest is achieved. According to the invention, a first fragment of the protein reporter is fused to a biological component of a cellular compartment of a given cell, whereas the complementary fragment of the protein reporter is fused to the virus of interest or to a transcription factor.

It is thus an object of the present invention to provide an immortal cell line that expresses a first fragment of a reporter protein in the nucleus of the cell and a modified transcription factor comprising a transcription factor fused to a second fragment of the reporter protein, said second fragment being complementary to the first fragment, wherein the modified transcription factor is able to mediate the innate immune response in the cells.

In a particular embodiment, the first fragment of the reporter protein is fused to a protein or motif conferring localization to the nucleus, such as SV40, or to a protein of the nuclear pore complex, such as a nucleoporin, preferably

Nup214, Nup98 or Nup153.

Advantageously, the first fragment of the reporter protein is fused to the protein of the nucleus via a flexible linker sequence and/or the second fragment of the reporter protein is fused to an interferon regulatory factor (IRF), a STAT protein or a subunit of NF-κB.

In a particular embodiment, the reporter protein is selected from the group consisting of a fluorescent protein, preferably a GFP-like fluorescent protein, and bioluminescent protein, preferably a NanoLuc-like protein.

Preferably, the first fragment is α-NanoLuc with the amino acid sequence set forth in SEQ ID NO: 2 and the second fragment is Cen-NanoLuc with the amino acid sequence set forth in SEQ ID NO: 3.

It is another object of the present invention to provide a process for monitoring activation of innate immunity signaling pathways in a population of cells comprising:

- Providing cells of the immortal cell line as described above;
- Subjecting said cells to a stimulus suitable to activate innate immunity signaling pathways, and
- Detecting the reconstituted reporter protein in the cells, subsequent to translocation of the transcription factor of interest in the nucleus;

wherein detection of the reconstituted reporter protein in the cells is indicative of the activation of the innate immunity signaling pathways in the cells.

Said process is particularly useful for screening candidate molecules able to stimulate and/or modulate innate immunity, wherein the stimulus consists in the candidate molecule(s) to be tested, wherein the detection of the reconstituted reporter protein in the cells is indicative that the candidate molecule is able to stimulate and/or to modulate the innate immunity.

Said process is also useful for screening candidate molecules able to overstimulate innate immunity, wherein cells are submitted to a stimulus suitable to activate the innate immunity and to the candidate molecule(s) to be tested (before, during or after submitting the cells to the stimulus activating the innate immunity), wherein an increase of detection of the reconstituted reporter protein in the cells compared to reconstituted reporter protein in control cells, is indicative that the candidate molecule is able to over-activate the innate immunity.

Said process is also useful for screening candidate molecules able to inhibit innate immunity, wherein the cells are submitted to a stimulus suitable to activate the innate immunity and to the candidate molecule(s) to be tested (before, during or after submitting the cells to the stimulus activating the innate immunity), wherein an absence of detection of the reconstituted reporter protein in the cells, or a decrease of detection of the reconstituted reporter protein in the cells compared to reconstituted reporter protein in control cells, is indicative that the candidate molecule is able to inhibit stimulation of the innate immunity.

It is a further object of the present invention to provide a kit for screening candidate molecules acting on innate immunity pathway, said kit comprising at least one immortal cell line as described above, and optionally one or more agonist(s) of interferon and inflammation signaling adapted to the cell line provided in the kit.

It is another object of the present invention to provide a process for monitoring the viral translocation of a virus of interest to a subcellular component of interest in a population of cells comprising:

- Providing cells that express a first fragment of a reporter protein tethered to the subcellular component of interest;
- Providing a virus of interest, wherein at least one viral protein has been tagged with second fragment of the reporter protein, said second fragment being complementary to the first fragment,
- Subjecting said cells to said virus;
- Detecting the reconstituted reporter protein in the cell;

wherein detection of reconstituted reporter protein in the cell is indicative of the viral translocation of the viral protein to said subcellular component.

Said process can be used for screening antiviral candidate molecules, wherein the cells are subjected both to the virus and to at least one antiviral candidate molecule, wherein an absence of detection of the reconstituted reporter protein in the cells or a decrease of detection of the reconstituted reporter protein in the cells compared to reconstituted reporter protein in control cells, is indicative that the candidate molecule is able to inhibit the viral infection.

The present invention further relates to the use of a split reporter protein for monitoring and/or evaluating and/or quantifying the viral trafficking of a virus of interest between subcellular compartments in a cell population, wherein a

first fragment of the reporter protein is fused to a protein of a subcellular compartment of interest in the cell population, and the second fragment of the reporter protein is fused to a viral protein of the virus of interest.

In a particular embodiment of said process or use, the reporter protein is selected from the group consisting of a fluorescent protein, preferably a GFP-like fluorescent protein, and bioluminescent protein, preferably a NanoLuc-like protein.

In a particular embodiment of said process or use, the first fragment is α-NanoLuc with the amino acid sequence set forth in SEQ ID NO: 2 and the second fragment is Cen-NanoLuc with the amino acid sequence set forth in SEQ ID NO: 3.

In a particular embodiment of said process or use, the first fragment of the reporter protein is fused to a protein or motif conferring localization to a subcellular compartment selected from the group consisting of the nucleus, the endoplasmic reticulum, the nuclear pore complex or the mitochondria and/or wherein the second fragment of the reporter protein is fused to an integrase protein of the virus.

It is a further object of the present invention to provide a kit comprising

- an immortal cell line that expresses a first fragment of a reporter protein in a subcellular compartment of interest, and
- a virus wherein at least one viral protein has been tagged with a second fragment of the reporter protein, said second fragment being complementary to the first fragment; and optionally
- a substrate for the protein reporter (e.g. fumirazine).

## Brief description of the drawings

**Figure 1. Principle of the αCentauri protein complementation assay and constructs for viral trafficking, a.** Schematic drawing of the αCentauri protein complementation assay showing the two fragments of a fluorescent or luminescent reporter. The small fragment α is tagged on a relevant viral protein, while the larger Centauri fragment (Cen) is fused to a subcellular compartment. Upon viral trafficking, the α and Centauri fragments are brought into close proximity and assemble to form functional α Centauri reporter. b. Schematic drawing showing the application of αCentauri in monitoring HIV-1 docking at the nuclear envelope and entry into the nucleus. The small α fragment is tagged to HIV-1 integrase (IN), while the larger Centauri (Cen) fragment is fused either to Nup214 or to a nuclear localization signal (NLS) for targeting to the nuclear pore or to the nucleus, respectively. c. Schematic drawing of the genomic organization of the HIV-1 constructs indicating the insertion of $\alpha^{GFP}$ or $\alpha^{NLuc}$ in C-ter of viral IN. The genome is represented to-scale using Illustrator for Biological Sequences (IBS). The 225T central polypurine tract (cPPT) mutant and D116I IN mutant are indicated. All experiments use Δenv VSVG pseudotyped HIV-1 unless otherwise indicated. LTR, long-terminal repeat. **d.** Graph showing the impact of the α insertion on virus production. HEK 293T cells were transiently transfected with full-length proviral expression plasmids (HIV, $\alpha HIV^{GFP}$ or $\alpha HIV^{NLuc}$). Virus production was measured by quantitation of p24 viral antigen in cell supernatants at 48 hours post-transfection. Graphs show individual values from four independent experiments. Ordinary one-way ANOVA was performed using Prism 6; ns= non-significant (p=0.0540). **e.** Graph showing the impact of the α insertion on infectivity. Single cycle virus titrations were carried out in P4 cells. β-galactosidase activity was measured by chemiluminescent assay at 48 hours post-infection (hpi). Results are expressed as relative light units (RLU)/s/ng p24 of the inoculum, mean SD of four independent experiments. Two-tailed paired t test was performed using Prism 6; ns= non-significant (p=0.5579 for $\alpha HIV^{GFP}$ and p=0.2253 for $\alpha HIVN^{Luc}$). **f.** Schematic representation of the $Cen^{NLS}$ and $Cen^{Nup214}$ constructs. Complementary fragments of the superfolder GFP ($Cen^{GFP}$) or Nanoluciferase ($Cen^{NLuc}$) were cloned upstream of a HA-tag followed by a SV40 NLS or the full-length sequence of Nup214. **g.** Microscope image showing localisation of $Cen^{NLS}$ and $Cen^{Nup214}$. Centauri constructs were ectopically expressed in HeLa cells by lentiviral transduction (NLS constructs) or plasmid transfection (Nup214). Localisation was assessed by indirect HA-immunolabelling at 48 hours post-transduction (hpt) or 24 hours post-transfection. Scale bar = 10μm.

**Figure 2. Assessment of HIV-1 trafficking by fluorescent $\alpha Centauri^{GFP}$ assay. a.** Microscope image of HIV-1 trafficking to the nuclear generating αCentauri signal. HeLa cells transfected with $CenNup214^{GFP}$ were infected with $\alpha HIV^{GFP}$ (VSV-G pseudotyped for all panels of Fig. 2) for 30 min. Images were acquired on an Airyscan LSM880 microscope. $CenNup214^{GFP}$ was detected by HA labelling, and HIV-1 capsid (CA) by labelling with AG3.0 monoclonal antibody. Arrows point to $\alpha Centauri^{GFP}$ spots, while insets show representative zoomed in images from two independent experiments. Scale bar = 5μm. **b.** Microscope images of HIV-1 trafficking to the nucleus generating αCentauri signal. HeLa cells transduced with $CenNLS^{GFP}$ were infected with $aHIV^{GFP}$. αCentauri signal was imaged at 48 hpi using LSM700 confocal microscope. Images are representative from four independent experiments. Nuclear and

cytoplasmic GFP signal was quantified using ImageJ on a total of 40 cells from 7 independent fields. Scale bar = 10μm. **c.** Flow cytometry graphs and plots showing αCentauri$^{GFP}$ complementation in different cell types. Efficient transduction with CenNLS$^{GFP}$ was assessed by indirect immunofluorescence labelling of the HA tag followed by flow cytometry (left histograms). Cells were then infected with αHIV$^{GFP}$ or left uninfected (ni), and sfGFP signal was assessed by flow cytometry at 48 hpi (dot plots). Plots show representative experiments while graphs show individual values +/- SEM from three independent experiments for each cell type. Panels d-g are HeLa cells. **d.** Flow cytometry graphs showing the effect of Aphidicolin (APH) and Nevirapine (NVP) on αCentauri$^{GFP}$. CenNLS$^{GFP}$ cells were treated with APH, NVP or DMSO, and infected with αHIV$^{GFP}$. GFP complementation was assessed at 48 hpi by flow cytometry. Cell cycle block in G0/G1 was assessed by propidium iodide labelling (PI) (right histograms). Panels are representative of two independent experiments. **e.** Flow cytometry graphs and plots showing the assessment of a HIV-1 nuclear import mutant on αCentauri complementation. CenNLS$^{GFP}$ cells were infected with αHIV$^{GFP}$ or αHIV-225T$^{GFP}$. α Centauri complementation was measured at 48 hpi by flow cytometry. Plots are representative of three independent experiments, while the left-hand graph shows individual GFP values from three independent experiments with mean +/- SEM. Viral nuclear import was assessed by qPCR quantification of 2-LTR circles normalized for late reverse transcripts (POL). The ratio of 2LTR/POL copy numbers was 0.01 to 0.5 for αHIV$^{GFP}$ across experiments. Results are normalized for αHIV$^{GFP}$. The right-hand graph shows individual values from 3 independent experiments +/- SD. **f.** Flow cytometry graphs showing the time course of αCentauri$^{GFP}$ complementation. CenNLS$^{GFP}$ cells were infected with αHIV$^{GFP}$ and fluorescent signal was measured at 6, 12, 24, 30 and 48 hpi. Results are representative of two independent experiments. **g.** Flow cytometry graphs and plots showing the frequency of αCentauri complementation in productively infected cells. CenNLS$^{GFP}$ cells were infected with untagged HIV-1 or αHIV, or left uninfected (ni). At 48 hpi, productive infection was assessed by indirect immunofluorescence labelling of intracellular Gag using KC67 antibody. Plots show one representative experiment, while the graph shows mean 95 CI for 4 independent experiments.

**Figure 3. Assessment of HIV-1 trafficking by luminescent αCentauri$^{NLuc}$ assay. a.** Flow cytometry graphs showing αCentauri$^{NLuc}$ complementation in different cell types. Efficient transduction with CenNLS$^{NLuc}$ was assessed by indirect immunofluorescence labelling of the HA tag followed by flow cytometry (left histograms). Cells were then infected with αHIV$^{NLuc}$ or left uninfected (ni), and NLuc signal was assessed as relative light units per second (RLU/s) by plate luminometry at 24 hpi. Graphs show individual values +/- SEM from 2 independent experiments. All subsequent panels are HeLa cells. **b.** Flow cytometry graphs and plots showing that αCentauri$^{NLuc}$ signal is related to the efficiency of Cen$^{NLuc}$ expression. CenNLS$^{NLuc}$ cell clones were characterized by HA-labelling. Cytometry plots are representative of four independent labelling experiments. The measurement of transduction efficiency is provided as the product of the geometric mean fluorescence and percentage HA+ cells. Clones were then infected with αHIV$^{NLuc}$ or with untagged HIV-1, and NLuc complementation was assessed after 24h. The graph shows individual values and mean +/- SD from 2 independent experiments. **c.** Flow cytometry graphs showing the time course of αCentauri$^{NLuc}$ complementation. CenNLS$^{NLuc}$ cells were infected with αHIV$^{NLuc}$ and luminescent signal was measured at 16, 24, 36 hpi and represented as fold signal (RLU/s) above uninfected background control. Results show all values from 3 independent experiments as box and whisker plots. **d.** Histogram showing αCentauri assay using wild-type envelope HIV-1. CenNLS$^{NLuc}$ P4 cells were infected with αHIVNLuc (WTenv) and luminescent signal was measured at 24 hpi. Results show RLU/s values from two independent experiments, **e.** Graph showing that αCentauri complementation in the nucleus correlates with HIV-1 genome nuclear import. CenNLS$^{NLuc}$ cells were infected with different doses of αHIV$^{NLuc}$. Viral nuclear import was assessed by qPCR quantification by the ratio of 2-LTR circles over total reverse transcribed HIV (POL). αCentauri was measured at 24 hpi for each condition. The graph shows individual values from 3 independent CenNLS$^{NLuc}$ clones. **f.** Graphs showing that integration-defective HIV-1 generates robust αCentauri complementation. CenNLS$^{NLuc}$ cells were infected with 2 POL copies/cell of αHIV, αHIV with NVP, αHIV-D116I$^{NLuc}$ or untagged HIV-1 for 24h. The left graph shows individual values (as fold increase in RLU/s relative to untagged HIV-1) from 3 independent experiments performed on a total of 12 Cen$^{NLuc}$ HeLa cell lines, and median with interquartile range. Statistical analysis was performed by unpaired t test using Prism 6. ***, p=0.0002. HIV-1 integration was assessed by qPCR quantification of Alu-HIV segments and reverse transcribed HIV (late reverse transcripts). Results are normalized for αHIV$^{NLuc}$. The right-hand graph shows individual values from 3 independent CenNLS$^{NLuc}$ clones +/- SD. **g.** Graphs showing that αCentauri$^{NLuc}$ allows quantification of HIV-1 docking at nuclear pores. HEK 293T cells were transfected with CenNup214 by calcium phosphate coprecipitation, then seeded in 96-well plate and infected with αHIV$^{NLuc}$. NLuc signal was detected at 6 hpi. Results show independent values from 2 independent experiments.

**Figure 4. Benchmarking and quality control of αCentauri towards screening. a.** Graphs showing a comparison of αCentauri with alternative assays of HIV-1 replication used for screening. Four HIV infection reporter systems were tested for read-out at different transducing units (TU) per cell. **First graph**: HeLa cells were transduced with

CenNLS$^{GFP}$ then infected with $\alpha$HIV$^{GFP}$ at the indicated TU per cell. The graph plots the percentage of GFP-positive cells at 48 hpi assessed by flow cytometry, and shows individual values from four independent experiments with a hyperbola curve fit ($R^2$=0.93). **Second graph**: HeLa-CenNLSNLuc cells were infected with $\alpha$HIV$^{NLuc}$ at the indicated TU/cell. The graph plots the relative increase in RLU/s at 24 hpi (as fold relative to untagged HIV-1) and shows individual values from four independent experiments with a linear regression ($R^2$=0.91). **Thrid graph**: Hela-LTR-LacZ cells were infected with HIV-1 at the indicated TU/cell. The $\beta$-gal signal was measured at 48 hpi using a chemiluminescent assay kit from Roche according to manufacturer's instructions and normalized for protein content measured by Bradford assay. Individual values 102 from 2 independent experiments are shown with a second-order polynomial fit ($R^2$=0.89). **Fourth graph:** HeLa cells were infected with HIV-1 at the indicated TU/ml. At 48 hpi, cells were fixed and labelled with an anti-gag KC67 antibody to assess the percentage of infected cells by flow cytometry. The graph shows a single representative experiment with a hyperbola curve fit ($R^2$=0.99). For each graph, min/max values show the fold signal between the highest and lowest infectious dose for each assay. **b.** Graph showing a comparison of different fumirazine substrates with commercial Nano-Glo (nGlo). CenNLS$^{NLuc}$ HeLa cells were infected with $\alpha$HIV. At 24 hpi, substrate was added directly to each well with a 1:1 ratio. Luminescence was measured 3min after substrate addition. Final substrate concentration was 40-50$\mu$M, except for Nano-Glo where the stock concentration was not provided by the manufacturer (Promega). The graph shows individual values from 2 independent experiments. **c.** Graph showing quality control distribution of positive and negative controls. Z' factors were calculated for $\alpha$Centauri$^{GFP}$ using $\alpha$HIV$^{GFP}$ versus $\alpha$HIV$^{GFP}$ with Nevirapine (NVP), and for $\alpha$Centauri$^{NLuc}$ using $\alpha$HIV$^{NLuc}$ or $\alpha$HIVD116I$^{NLuc}$ versus $\alpha$HIV$^{NLuc}$ + NVP. Examples are representative of three independent experiments. **d.** Graphs showing shRNA-based screen of cellular cofactors of HIV-1 nuclear import using $\alpha$Centauri. HeLa CenNLS$^{NLuc}$ stable clones were transduced with lentiviral vectors coding for the indicated shRNAs for 2 days then infected with $\alpha$HIV$^{NLuc}$ or untagged HIV-1 at 50 Pol copies/20,000 cells and $\alpha$Centauri signal was measured at 24 hpi. NLuc signal (RLU/s) was normalized for protein content in each well by Bradford assay, and values for $\alpha$HIV$^{NLuc}$ were normalized as fold-values over background values obtained with untagged HIV-1. The graph shows a box and whisker plot of 2 independent experiments performed in duplicate. Right-hand graphs show levels of knockdown obtained by qPCR analysis of the indicated transcripts, as individual values from experimental replicates and mean +/- SD.

**Figure 5. Principle of the $\alpha$Centauri protein complementation assay and constructs for quantification of nuclear translocation of transcription factors involved in innate immunity. a.** Schematic drawing showing the application of $\alpha$Centauri in monitoring innate immunity signaling involving nuclear translocation of transcription factors (TF) of innate immune signaling pathways. The small $\alpha$ fragment is tagged to Flag-TF while the larger Centauri (Cen) fragment is fused to a nuclear localization signal (NLS) for targeting to the nucleus. The PCA is completed when a virus, or bacteria, infects the cell leading to the phosphorylation and translocation of pTF into the nucleus. **b.** Example showing the application of $\alpha$Centauri in monitoring innate immunity signaling involving nuclear translocation of IRF3. The small $\alpha$ fragment is tagged to Flag-IRF3 while the larger Centauri (Cen) fragment is fused to a nuclear localization signal (NLS) for targeting to the nucleus. The PCA is completed when a virus, for instance Sendai virus (SeV), infects the cell leading to the phosphorylation and translocation of pIRF3 into the nucleus. **c.** Example showing the application of $\alpha$Centauri in monitoring innate immunity signaling involving nuclear translocation of p65. The small $\alpha$ fragment is tagged to the sub-unit p65 of NF-$\kappa$B while the larger Centauri (Cen) fragment is fused to a nuclear localization signal (NLS) for targeting to the nucleus. The PCA is completed after exposure of the cell to a lipopolysaccharide (LPS) stimulus and activation of TLR4 receptors, leading to the translocation of NK-$\kappa$B into the nucleus.

**Figure 6. Assessment of IRF3 trafficking by fluorescent $\alpha$Centauri$^{NLuc}$ assay. a.** Diagram of an $\alpha$Cen complementation experiment using IRF3 as proof-of-concept. This is carried out either by co-transfection of plasmids encoding the partners, pCDNA3.1 (+) IRF3$\alpha$-3xFLAG and pCDNA3.1 (+) Cen-HA-NLS, into HEK293T cells (light gray), or by transduction using a lentiviral vector coding for IRF3$\alpha$-3xFLAG in a HeLa clone stably expressing Cen-HA-NLS (named E4, in dark gray). The nuclear translocation of IRF3$\alpha$ is induced by six hours of infection with Sendai virus (SeV). The cells thus infected or not are placed in a 96-well plate and the substrate from the NanoGlo Luciferase Assay kit (Promega) is added according to the manufacturer's instructions. The luminescence is then measured by a TECAN Infinite 200 microplate reader. **b.** Graphs showing $\alpha$Cen complementation in HEK293T as shown in light gray (a). On the left is shown the luminescence intensity in RLU of a representative experiment, emitted after addition of the substrate. NI = Not Infected / I = Infected. On the right is presented the quantitative difference in $\alpha$Cen complementation intensity in arbitrary units (n = 2). **c.** Graphs showing $\alpha$Cen complementation in the stable HeLa E4 Cen-HA-NLS clone as shown in dark gray (a). On the left is shown the luminescence intensity in RLU of a representative experiment, emitted after addition of the substrate. On the right is presented the quantitative difference in $\alpha$Cen recomplementation intensity in arbitrary units (n = 2). **d.** RT-qPCR measurement of the induction

of IFN-β in the HeLa E4 clone Cen-HA-NLS in representative αCen recomplementation experiments (n = 2). NT = Not Transduced. T = Transduced. The results were normalized for the housekeeping gene RPL13A. **e.** Immunofluorescence of the αCen complementation in the stable HeLa E4 Cen-HA-NLS clone after infection or not for 6 hours with SeV, visualized Airyscan LSM880 confocal microscope (Zeiss). IRF3α (in magenta) is labeled with a mouse monoclonal anti-Flag M2 primary antibody (Sigma) diluted to the final 1/2000, followed by an Alexa 488 secondary anti-mouse antibody (Probes Invitrogen) diluted to 1/1000. Cen-HA-NLS (in green) is labeled with an anti-HA mouse antibody coupled to Alexa 647 (6E2, Cell Signaling Technology) diluted 1/100. **f.** Bioluminescence microscopy images showing αCen complementation in the stable HeLa E4 Cen-HA-NLS clone after infection or not for 6 hours with SeV.

**Figure 7. Comparison of Alpha-Centauri with alternative available systems to quantify innate signalling. a.** Alpha Centauri detects IRF-3 translocation earlier than any other system, since it is not dependent on gene expression for its read-out. The recommended use for ISRE-Luc and SEAP systems is at 24 hours post-stimulation (hps) to allow for signal transduction, transcription and translation to occur. Since the Alpha-Centauri system relies only on protein nuclear translocation, it may be used at very early time points. Results shown are at 6 hps, but luminescence was detected as early as 15 min post-stimulation with TNF in the case of the Alpha-Centauri NF-κB system (data not shown). Cells were transfected with a constitutively active form of RIG-I (2CARD plasmid, 40ng), or treated with 5μg/ml 2'3'-cGAMP, a non-canonical cyclic dinucleotide (this is the positive control for the SEAP kit according to manufacturer's protocol), 1μg/ml doxorubicin, Sendai virus (SeV), or lipopolysaccharide (LPS). At 6hps, results were entirely negative for SEAP cells (despite several hours incubation with the substrate, and even with the positive control 2'-3'-cGAMP). In the case of ISRE-Luc, only SeV and 2'-3'-cGAMP produced positive read-outs, whereas all stimuli were detected by the Alpha-Centauri system. **b.** False positives detected by ISRE-Luc and SEAP are not detected by Alpha Centauri. Cells were pre-treated with 5 mM Valproate, a HDAC inhibitor, for 1h, then infected with Sendai virus 1/1000e. Results are at 6 hours post-stimulation (6hps) for Alpha-Centauri and 24hps for ISRE-Luc and SEAP. Valproate activates gene expression non-specifically and therefore creates signal in both ISRE and SEAP systems. Of note, the absorbance values obtained for Valproate in the SEAP (Invivogen) system were the same as those obtained for 2'3'-cGAMP (the positive control). In contrast, Valproate did not generate any signal in the Alpha-Centauri system. False positive= valproate. Real positives: DD778 (pyrimidine biosynthesis inhibitor, see Lucas-Hourani et al., 2017), 2'3'-cGAMP (kit positive control), and Sendai virus. **c.** False negatives not detected by ISRE-Luc and SEAP are detected by Alpha Centauri. Cells were treated with LPS, 5μM DD778, or with 1μg/ml poly I:C in the culture medium. Results are at 6 hours post-stimulation (6hps) for Alpha-Centauri and 24hps for ISRE-Luc and SEAP. ISRE-Luc cells are unresponsive to TLR4 agonists such as LPS, SEAP cells are unresponsive to pyrimidine biosynthesis inhibitors, but Alpha Centauri detects both. False negatives= LPS, DD778 (pyrimidine biosynthesis inhibitor, see Lucas-Hourani et al., 2017). Real negative: non-transfected poly I:C.

**Figure 8. Schematic drawing summarizing αCentauri PCA in monitoring nuclear translocation of a virus, an IRF transcription factor and NF-κB.** Schematic drawing of the αCentauri protein complementation assay showing the two fragments of a fluorescent or luminescent reporter. The small fragment α is tagged on a relevant viral protein, an IRF transcription factor and NF-κB, while the larger Centauri fragment (Cen) is fused to a nuclear localization signal for targeting to the nucleus. Upon viral trafficking, or nuclear translocation of IRF transcription factor or NF-κB, the α and Centauri fragments are brought into close proximity and assemble to form functional α Centauri reporter.

## Detailed description of the invention

*Definition*

As used herein, a "protein reporter" refers to a polypeptide molecule that can be detected in cells by ordinary means, such as spectroscopic means (e.g. fluorometry, mass spectrometry) or biochemical means (e.g. enzymatic reactions). For example, a fluorescent protein and the like (e.g. a green fluorescent protein (GFP) or the like) can be used as a protein reporter. Alternatively, a bioluminescent protein, such as luciferase, nanoluciferase or the like can be used as a protein reporter.

As used herein, the term "bioluminescence" refers to production or emission of light by a reaction catalyzed by, or enabled by, an enzyme, protein, protein complex, etc. In typical embodiments, a substrate for bioluminescent entity is converted into unstable form by the bioluminescent entity. The substrate subsequently emits a bioluminescent signal (i.e. light) that can be detected/measured/monitored.

As used herein, "complementary fragment(s)" when used in reference to a protein reporter refer to fragments of a protein reporter that are individually inactive (i.e., do not express the reporter phenotype), wherein binding of the complementing fragments restores reporter activity.

The terms "subcellular compartment" refers to various distinguishable part, components or organelles of a cell, including without limitation, the nucleus, cytoplasm, plasma membrane, endoplasmic reticulum, Golgi apparatus, endosome, peroxisome and mitochondria.

The terms "fused" or "tethered" are used interchangeably and refer to linkage by covalent bonding.

The term "linker" refers to a molecule or group of molecules that connects two molecules, such as a fragment of a protein reporter and a protein or nucleic acid.

Protein reporter

The present invention is based on the use of a protein reporter and more particularly of a protein reporter that is split into two complementary fragments, which are linked to different compartments of a given cell and/or to a given virus, to study the intracellular trafficking of components of interest. Complementation of the protein reporter is indicative of trafficking of a protein or virus of interest within a given subcellular compartment of the cell.

Any protein reporter able to emit a luminescent signal can be used. In a particular embodiment, the protein reporter is a bioluminescent protein, such as a luciferase and nanoluciferase (NanoLuc), or the like. In another embodiment, the protein reporter is a fluorescent protein or the like, such as a GFP and GFP-like protein.

According to the invention, the protein reporter is split into two complementary fragments, which are each linked to a given protein located in a subcellular compartment or to a viral protein. Advantageously, the protein reporter is split into two fragments of unequal size, i.e. into a small fragment and a large fragment. The fragments are advantageously folded and soluble in the cellular environment. Advantageously, the small fragment is small enough to not perturb the solubility of the fused protein and/or to minimize the potential for interference with cellular processing and transport of the fusion protein. Unless otherwise specified, in the context of the invention, the protein reporter is split into two complementary fragments, called either 1st fragment and second fragment, or large fragment and small fragment.

Advantageously, the large fragment is fused to a protein expressed in a subcellular compartment of interest, whereas the small fragment is fused to a protein whom trafficking is studied (e.g. transcription factor, viral protein).

In a particular embodiment, the protein reporter is a nanoluciferase (NanoLuc) split into a small fragment, called α fragment, and a large fragment, called Centauri fragment (or Cen). The amino acid sequence of NanoLuc is

SEQ ID NO: 1 (GenBank: AFI79290.1):

MVFTLEDFVGDWEQTAAYNLDQVLEQGGVSSLLQNLAVSVTPIQRIVRSGENALKID
IHVIIPYEGLSADQMAQIEEVFKVVYPVDDHHFKVILPYGTLVIDGVTPNMLNYFGRP
YEGIAVFDGKKITVTGTLWNGNKIIDERLITPDGSMLFRVTINGVTGWRLCERILA

wherein the α fragment corresponds to SEQ ID NO: 2: GVTGWRLCERILA
and the Centauri fragment corresponds to SEQ ID NO: 3

MVFTLEDFVGDWEQTAAYNLDQVLEQGGVSSLLQNLAVSVTPIQRIVRSGENALKID
IHVIIPYEGLSADQMAQIEEVFKVVYPVDDHHFKVILPYGTLVIDGVTPNMLNYFGRP
YEGIAVFDGKKITVTGTLWNGNKIIDERLITPDGSMLFRVTIN.

In another particular embodiment, the protein reporter is a GFP split into a small fragment, called α fragment, and a large fragment, called Centauri fragment (or Cen). The amino acid sequence of GFP is

SEQ ID NO: 4

MVSKGEELFTGVVPILVELDGDVNGHKFSVRGEGEGDATIGKLTLKFICTTGKLPVP
WPTLVTTLTYGVQCFSRYPDHMKRHDFFKSAMPEGYVQERTISFKDDGKYKTRAVV
KFEGDTLVNRIELKGTDFKEDGNILGHKLEYNFNSHNVYITANKQKNGIKANFTVRH
NVEDGSVQLADHYQQNTPIGDGPVLLPDNHYLSTQTVLSKDPNEKRDHMVLHEYV
NAAGIT

wherein the $\alpha$ fragment corresponds to SEQ ID NO: 5: RDHMVLHEYVNAAGIT
and the Centauri fragment corresponds to SEQ ID NO: 6:

MVSKGEELFTGVVPILVELDGDVNGHKFSVRGEGEGDATIGKLTLKFICTTGKLPVP
WPTLVTTLTYGVQCFSRYPDHMKRHDFFKSAMPEGYVQERTISFKDDGKYKTRAVV
KFEGDTLVNRIELKGTDFKEDGNILGHKLEYNFNSHNVYITANKQKNGIKANFTVRH
NVEDGSVQLADHYQQNTPIGDGPVLLPDNHYLSTQTVLSKDPNEK

In a particular embodiment, the fragments of the protein reporter are each fused to a given protein via a peptidic linker. Advantageously, the linker consists in a small peptide, comprising at most 20, at most 15 or at most 10 amino acid residues. In a preferred embodiment, the peptidic linker comprises between 6 and 8 amino acid residues. In a particular embodiment, the linker comprises amino acid residues selected from small polar amino acid residues, such as Gly and Ala (allowing to confer flexibility to the linker).

According to a first aspect of the invention, the small fragment is fused to a viral protein of a virus of interest.

According to a second aspect of the invention, the small fragment is fused to a transcription factor. Preferably, the small fragment is fused to an interferon regulatory factor (IRF), such as IRF1, IRF3, IRF7, IRF9, a STAT protein, such as STAT1, or a subunit of NF-$\kappa$B, such as p65.

In an embodiment, the large fragment of the protein reporter is fused to a protein or motif conferring localization to the nucleus. In a particular embodiment, the large fragment of the protein reporter is fused to a protein of nuclear pore complex (e.g., any nucleoporin such as Nup214, Nup98, Nup153). In another particular embodiment, the large fragment of the protein reporter is fused to a protein carrying a nuclear localization protein (NLS) (e.g., SV40).

In an embodiment, the large fragment of the protein reporter is fused to a protein of endoplasmic reticulum (e.g. Rab proteins, calnexin).

In an embodiment, the large fragment of the protein reporter is fused to a protein of mitochondria (e.g. voltage-dependent anion channel VDAC, cytochrome c oxidase COX).

It is a further object of the invention to provide a nucleic acid encoding a recombinant protein comprising or consisting in a small fragment of a protein reporter fused to a viral protein or a transcription factor.

It is a further object of the invention to provide a nucleic acid encoding a recombinant protein comprising or consisting in a large fragment of a protein reporter fused to a protein or motif conferring localization to a subcellular compartment of interest such as a protein or motif conferring localization to the nucleus (e.g. a protein of nuclear pore complex, a protein carrying a nuclear localization protein) or a protein of endoplasmic reticulum, or a protein of mitochondria.

As used herein, the term *"nucleic acid", "nucleic sequence," "polynucleotide",* "*oligonucleotide*" and *"nucleotide sequence"* are used interchangeably and refer to a sequence of deoxyribonucleotides and/or ribonucleotides. The nucleic acids can be DNA (cDNA or gDNA), RNA, or a mixture of the two. It can be in single stranded form or in duplex form or a mixture of the two. It can be of recombinant, artificial and/or synthetic origin and it can comprise modified nucleotides, comprising for example a modified bond, a modified purine or pyrimidine base, or a modified sugar. The nucleic acids of the invention can be in isolated or purified form, and made, isolated and/or manipulated by techniques known per se in the art, e.g., enzymatic synthesis or recombinant technology. The nucleic acids can also be synthesized in vitro by well-known chemical synthesis techniques, as described in, e.g., Belousov (1997) Nucleic Acids Res. 25:3440-3444.

Nucleic acids of the invention may further comprise additional nucleotide sequences, such as regulatory regions, i.e., promoters, enhancers, silencers, terminators, signal peptides and the like that can be used to cause or regulate expression of the polypeptide in a selected host cell or system. Alternatively, or in addition, nucleic acids of the invention

may further comprise additional nucleotide sequences encoding fusion proteins, such as maltose binding protein (MBP) or glutathion S transferase (GST) that can be used to favor polypeptide expression and/or solubility.

The present invention further relates to an expression cassette comprising a nucleic acid according to the invention operably linked to one or more control sequences that direct the expression of said nucleic acid in a suitable host cell. As used herein, the term *"expression cassette"* denotes a nucleic acid construct comprising a coding region, i.e. a nucleic acid of the invention, and a regulatory region, i.e. comprising one or more control sequences, operably linked.

Typically, the expression cassette comprises, or consists of, a nucleic acid according to the invention operably linked to a control sequence such as transcriptional promoter and/or transcription terminator. The control sequence may include a promoter that is recognized by a host cell or an *in vitro* expression system for expression of a nucleic acid encoding a protease of the present invention. The promoter contains transcriptional control sequences that mediate the expression of the recombinant protein. The promoter may be any polynucleotide that shows transcriptional activity in the host cell including mutant, truncated, and hybrid promoters, and may be obtained from genes encoding extracellular or intracellular polypeptides either homologous or heterologous to the host cell. The control sequence may also be a transcription terminator, which is recognized by a host cell to terminate transcription. The terminator is operably linked to the 3'-terminus of the nucleic acid encoding the recombinant protein. Any terminator that is functional in the host cell may be used in the present invention. Typically, the expression cassette comprises, or consists of, a nucleic acid according to the invention operably linked to a transcriptional promoter and a transcription terminator.

The invention also relates to a vector comprising a nucleic acid or an expression cassette as defined above.

The term "*vector*" refers to DNA molecule used as a vehicle to transfer recombinant genetic material into a host cell. The major types of vectors are plasmids, bacteriophages, viruses, fosmids, cosmids, and artificial chromosomes. The vector itself is generally a DNA sequence that consists of an insert (a heterologous nucleic acid sequence, transgene) and a larger sequence that serves as the "backbone" of the vector. The purpose of a vector which transfers genetic information to the host is typically to isolate, multiply, or express the insert in the target cell. Vectors called expression vectors (expression constructs) are specifically adapted for the expression of the heterologous sequences in the target cell, and generally have a promoter sequence that drives expression of the heterologous sequences encoding a polypeptide. Generally, the regulatory elements that are present in an expression vector include a transcriptional promoter, a ribosome binding site, a terminator, and optionally present operator. Preferably, an expression vector also contains an origin of replication for autonomous replication in a host cell, a selectable marker, a limited number of useful restriction enzyme sites, and a potential for high copy number. Examples of expression vectors are cloning vectors, modified cloning vectors, specifically designed plasmids and viruses. Expression vectors providing suitable levels of polypeptide expression in different hosts are well known in the art. The choice of the vector will typically depend on the compatibility of the vector with the host cell into which the vector is to be introduced.

Virus trafficking

The recent emergence and re-emergence of viruses in the human population has highlighted the need for cell-based assays of viral replication. Viruses traffic between different subcellular compartments to replicate, including between the plasma membrane, endocytic vesicles, the cytoplasm, the nucleus and endoplasmic reticulum. It is the purpose of the present invention to provide protein complementation assay to measure viral trafficking between subcellular compartments. The present invention allows to quantify viral translocation between subcellular compartments by protein complementation assays. More particularly, a fragment of the protein reporter is tethered to a subcellular compartment of interest whereas the complementary fragment of said protein reporter is tethered to a viral protein of the virus to study. A translocation within the subcellular compartment of interest brings together the two complementary fragments that do not emit any signal alone, creating a binary gain-of-signal. The invention allows to quantify a specific step of viral replication.

Generally speaking, the present invention relates to the use of a split protein reporter for monitoring / evaluating / quantifying the viral trafficking of a virus of interest between subcellular compartments in a cell population, wherein a 1$^{st}$ fragment of the protein reporter is fused to a protein of a subcellular compartment of interest in the cell population, and the second fragment of the protein reporter is fused to a viral protein of the virus of interest.

The protein complementation assay (PCA) developed by the inventors is a highly quantitative read-out of viral replication based on the quantification of viral translocation between subcellular compartments by proximity-based PCA. Since all viruses traffic between different subcellular compartments to replicate, including between the plasma membrane, endocytic vesicles, the cytoplasm, the nucleus and endoplasmic reticulum, this is applicable to any virus.

The PCA developed by the inventors can be used for screening for antivirals and for fundamental research to better understand viral replication, etc.

*Cell line and recombinant virus*

It an object of the present invention to provide a recombinant cell that has been engineered to express a 1st fragment, preferably a large fragment, of a protein reporter tethered to a subcellular component of interest of said cell.

According to the invention, the 1st fragment of the protein reporter can be tethered to any subcellular component of the cell. Particularly, the 1st fragment of the protein reporter can be tethered to a protein selected from the group consisting of proteins of the nucleus, the endoplasmic reticulum, the nuclear pore complex, the mitochondria, etc.

In a particular embodiment, recombinant cells are selected from the group consisting in Hela cells, HEK 293T cells, HT-1080 cells, A549 cells, HCT116 cells, THP-1 cells, CEM cells, MT4 cells.

It is thus another object of the invention to provide a recombinant cell comprising a nucleic acid, an expression cassette or a vector as described above. The present invention thus relates to the use of a nucleic acid, expression cassette or vector according to the invention to transform, transfect or transduce a host cell. The choice of the vector will typically depend on the compatibility of the vector with the host cell into which it must be introduced.

According to the invention, the host cell may be transformed, transfected or transduced in a transient or stable manner. The expression cassette or vector of the invention is introduced into a host cell so that the cassette or vector is maintained as a chromosomal integrant or as a self-replicating extra-chromosomal vector. The term "host cell" also encompasses any progeny of a parent host cell that is not identical to the parent host cell due to mutations that occur during replication. The host cell may be any eukaryote cell useful in the production of a recombinant cell of the present invention.

The nucleic acid, expression cassette or expression vector according to the invention may be introduced into the host cell by any method known by the skilled person, such as electroporation, conjugation, transduction, competent cell transformation, protoplast transformation, protoplast fusion, biolistic "gene gun" transformation, PEG-mediated transformation, lipid-assisted transformation or transfection, chemically mediated transfection, lithium acetate-mediated transformation, liposome-mediated transformation.

It is another object of the present invention to provide a recombinant virus, whom trafficking must be studied, that has been engineered to express a second fragment, preferably a small fragment, of said protein reporter, tethered to a viral protein. Any internal viral protein can be tagged with said fragment of the protein reporter. It is thus another object of the invention to provide a recombinant virus comprising a nucleic acid, an expression cassette or a vector as described above. The present invention thus relates to the use of a nucleic acid, expression cassette or vector according to the invention to transform, transfect or transduce a virus.

Any virus may be used and engineered to express a fragment of the protein reporter. In a particular embodiment, the virus is selected from the group consisting in human immunodeficiency viruses (HIV), coronaviruses, such as β-coronaviruses, Influenza viruses, flaviviruses such as West Nile Virus and Usutu virus.

In a particular embodiment, the virus is a retrovirus and the small fragment of the protein reporter is tethered to an integrase protein of said virus.

*Monitoring the viral translocation of a virus*

The cells and virus described above may be used for monitoring the viral trafficking within cells as well as for screening for antiviral drug candidates.

It is an object of the present invention to provide a process for monitoring the viral translocation of a virus of interest to a subcellular component of interest in cells comprising:

- Providing recombinant cells that stably or transiently express a large fragment of a protein reporter tethered to said subcellular component of interest;

- Providing a recombinant virus of interest, wherein at least one viral protein has been tagged with a small fragment of the protein reporter, said small fragment being complementary to the large fragment,

- Contacting said cells with said virus in order to infect the cells;

- Detecting, and optionally quantifying, reconstituted protein reporter in the cell,

wherein detection of reconstituted protein reporter in the cell is indicative of the viral translocation of the virus to said subcellular component.

According to the invention, the protein reporter is reconstituted only if or when the virus reaches the subcellular component of interest within the cells. In a particular embodiment, the large fragment of the protein reporter is linked to a protein of the nucleus or the nuclear pore complex, in order to monitor the nuclear translocation of a viral protein.

In a particular embodiment, the protein reporter is a fluorescent protein, such as GFP or GFP-like.

In another particular embodiment, the protein reporter is a bioluminescent protein, such as NanoLuc or NanoLuc-like. In such case, the cells are subjected to a substrate (e.g. light-emitting compound such as luciferin, fumirazine or other coelenterazine analogues) prior to the step of detecting.

According to the invention, the detection of reconstituted protein reporters comprises or consists of the detection of a fluorescent or luminescent signal emitted when the protein reporter is reconstituted.

According to the invention, said signal may be further measured / quantified to evaluate with more accuracy the viral translocation to the target cellular compartment.

Such PCA may be further implemented for screening antiviral candidate molecules, able to prevent the viral translocation.

It is another object of the present invention to provide a process for screening antiviral candidate molecules, wherein the cells have been further contacted with at least one antiviral candidate molecule. An absence of detection of reconstituted protein reporter or a decrease of detection of reconstituted protein reporter compared to detection of reconstituted protein reporter for control cells (i.e. infected cells not submitted to said antiviral candidate molecule) is indicative that the candidate molecule has an antiviral activity.

Advantageously, the cells have been contacted with the candidate molecule before the step of detecting and/or quantifying the reconstituted protein reporter in the subcellular component of the cells. Alternatively, the cells are contacted simultaneously with the virus and the candidate molecule, or are contacted first with the virus and after with the candidate molecule.

This process for screening may be implemented with any virus and any candidate molecule.

*Kit for monitoring viral trafficking*

It is a further object of the present invention to provide a kit ready to use for monitoring viral trafficking between subcellular compartments of cells. According to the invention, such kit may comprise:

- an immortal cell line (i.e. recombinant cells as described above) that expresses a large fragment of a protein reporter fused to a protein expressed in a subcellular compartment of interest, and/or

- a recombinant virus wherein at least one viral protein has been tagged with a small fragment of the protein reporter.

Advantageously, the kit may further comprise a substrate for the protein reporter, such as an light-emitting compound (e.g. fumirazine).

Innate signaling

Activation of transcription factor NF-κB results in translocation of ubiquitously expressed NF-κB from the cytoplasm to the nucleus. NF-κB is associated with a number of diseases. There is thus an interest in identifying compounds that modulate or inhibit the nuclear translocation of activated NF-κB.

It is a purpose of the present invention to provide assays to detect the impact of a given test compound on NF-κB activity, or to screen for compounds able to modulate the activation of NF-κB pathway. The method of the present invention allows to identify and optionally quantify the nuclear translocation of NF-κB induced by the test compound(s) and thereby to determine the toxicity of the test compound(s) and their pro-inflammatory potential.

Generally speaking, the present invention relates to the use of a split protein reporter for monitoring and/or evaluating and/or quantifying the activation of innate immunity in cells.

More particularly, the present invention relates to the use of such split protein reporter for monitoring and/or evaluating and/or quantifying the nuclear translocation of transcription factors, such as interferon regulatory factors (IRF), STAT proteins or a subunit of NF-κB in a cell population. According to the invention, a large fragment of the protein reporter is fused to a protein of a subcellular compartment of interest in the cell population, and the small fragment of the protein reporter is fused to a transcription factor to monitor.

The protein complementation assay (PCA) developed by the inventors can be used for monitoring activation of innate immunity signaling pathways in a population of cells, as well as for screening candidate molecules able to modulate (e.g. activating, inhibiting) innate immunity in a population of cells, and the like.

*Cell line*

It is an object of the present invention to provide recombinant cells, and immortal cell lines, that have been engineered to express a 1st fragment, preferably a large fragment, of a protein reporter tethered to the nucleus of said cell.

According to the invention, the 1st fragment of the protein reporter can be tethered to any protein or motif conferring localization to the nucleus, such as a protein carrying a nuclear localization protein (NLS) (ex.: SV40) or a protein of the nuclear pore (ex.: nucleoporin Nup124).

And the second fragment of the protein reporter is tethered to a of transcription factors, such as interferon regulatory factors (IRF), STAT proteins or a subunit of NF-κB, such as p65.

The cells may be selected from any type of cell, including any type of human or non-human animal cell, including mouse cells or rat cells. The cells may be stem cells or may be somatic cells such as primary cells, established cell lines such as immortal or immortalized cells, tumour cells, germs cells or their precursors, as well as cells derived or differentiated from stem cells, including derived or differentiated from induced pluripotent stem cells.

In a particular embodiment, recombinant cells are selected from the group consisting in Hela cells, HEK 293T cells, HT-1080 cells, A549 cells, HCT116 cells, THP-1 cells, CEM cells, MT4 cells.

It is thus an object of the invention to provide a recombinant cell comprising nucleic acids, expression cassettes or vectors as described above allowing said cells to express the both recombinant proteins within the cells. Any method known in the art to transform, transfect or transduce in a transient or stable manner may be used.

*Monitoring activation of innate immunity signaling pathways*

The recombinant cells described above, expressing both the small fragment of the protein reporter tethered to a transcription factor able to mediate the innate immune response and the large fragment of the protein reporter tethered to a protein or motif of the nucleus can be used for monitoring the innate immunity signal pathway during infection.

It is thus an object of the present invention to provide a process for monitoring activation of innate immunity signaling pathways in a population of cells comprising:

- Providing recombinant cells as described above;
- Subjecting said cells to a stimulus suitable to activate innate immunity signaling pathways, and
- Detecting, and optionally quantifying, reconstituted protein reporter, subsequent to translocation of the transcription factor of interest in the nucleus

wherein detection of the reconstituted reporter protein in the cells is indicative of the activation of innate immunity signaling pathways in the cells.

According to the invention, the stimulus may be any extracellular stimulus able to infect the cells, such as, without limitation pathogens (e.g. viruses, bacteria), or to stimulate inflammation pathways, such as lipopolysaccharide (LPS).

According to the invention, the protein reporter is reconstituted only if or when the innate immunity signaling pathway of interest is activated and the corresponding tagged transcription factor reaches within the nucleus of the cells.

In a particular embodiment, the protein reporter is a fluorescent protein, such as GFP or GFP-like.

In another particular embodiment, the protein reporter is a bioluminescent protein, such as NanoLuc or NanoLuc-like. In such case, the cells are subjected to a substrate (e.g. light-emitting compound such as luciferin, fumirazine or other coelenterazine analogues) prior to the step of detecting.

According to the invention, the detection of reconstituted protein reporters consists in the detection of a fluorescent or luminescent signal emitted when the protein reporter is reconstituted.

According to the invention, said signal may be further measured / quantified to evaluate with more accuracy the activation of the innate immunity pathway.

The PCA developed by the inventors may be used for screening candidate molecule, either for evaluating their toxicity or their ability to enhance the innate immunity response.

To this end, the recombinant cells of the invention are contacted with the candidate molecule. The contacting may be done by adding the candidate molecule to the culture medium in which the cells are cultured. For example, the candidate molecule may be dissolved or dispersed in a liquid vehicle, such as a solvent or solution. The contacting may be done over a period of time, for example by incubating the candidate molecule that is to be tested with the cells in culture.

The concentration of the candidate molecule to be used may be varied, and may depend on the compound that is to be tested.

The candidate molecule may be any compound that is expected to come into contact with a subject, including being inhaled by, topically applied to, absorbed by, ingested by, administered to, or implanted into a subject. For example, the test compound may be a pharmaceutical compound, an organic compound, an inorganic compound, a pesticide, a herbicide, an environmental toxin, a fungal toxin, a microbial toxin, a heavy metal-containing compound, an organic solvent, a cleaning agent, a preservative, a food additive, a dietary supplement, a herbal compound, an animal derived

compound, an anti-microbial compound, a cosmetic ingredient, a microparticle or a nanoparticle.

In an embodiment, such PCA is used for screening candidate molecules able to stimulate and/or modulate innate immunity. To this end, the candidate molecule is used as the external stimulus in the above described process. In such case, detection of the reconstituted reporter protein in the cells is indicative that the candidate molecule is able to stimulate the innate immunity.

Alternative, the above described process further comprises the step of submitting the cells to a candidate molecule, before, after or simultaneously subjecting the cells to the external stimulus, in order to evaluate the impact of the candidate molecule on an already activated innate immunity pathway (i.e. the ability of the candidate compound to modulate the innate immunity). In a particular embodiment, the amount of reconstituted protein reporter can be compared to the amount in negative control cells (subjected to the extern stimulus but not to the candidate molecule) to evaluate with more accuracy the ability of the candidate molecule to trigger the studied pathway.

In another embodiment, such PCA may be used for screening candidate molecules able to inhibit innate immunity. To this end, the above described process further comprises the step of submitting the cells to a candidate molecule, before, after or simultaneously subjecting the cells to the external stimulus. In such case, absence of detection of the reconstituted reporter protein in the cells is indicative that the candidate molecule is able to inhibit stimulation of the innate immunity. In a particular embodiment, the amount of reconstituted protein reporter can be compared to the amount in negative control cells (subjected to the external stimulus but not to the candidate molecule) to evaluate with more accuracy the ability of the candidate molecule to inhibit the studied pathway.

The negative control cells, although not contacted with the candidate molecule, may be contacted with a negative control solution, for example the solvent or solution used to dissolve or disperse the candidate molecule.

*Kit for monitoring innate immunity pathways*

It is a further object of the present invention to provide a kit ready to use for monitoring innate immunity pathways in cells. According to the invention, such kit may comprise an immortal cell line (i.e. recombinant cells as described above) that expresses a large fragment of a protein reporter fused to a protein expressed in the nucleus and a small fragment of said protein reporter tethered to a transcription factor of interest, and optionally a substrate for the protein reporter, such as an light-emitting compound (e.g. fumirazine) and/or an agonist of interferon and inflammation signaling adapted to the cell line provided in the kit.

The kit can further comprise candidate molecules and optionally a positive or a negative control.

EXAMPLES

**Viral trafficking**

Material & Methods

*Cells and drugs*

P4 TAR-β-gal indicator cells are HeLa CD4+ CXCR4+ CCR5+ carrying the LacZ gene under the control of the HIV-1 LTR promoter (AIDS Reagent Program). HEK 293T (CRL-11268), Hela (CCL-2) and A549 (CCL-185) were obtained from the ATCC. The MT4R5 [1] and CEM CD4+ (NIH 117) T cell lines were grown in RPMI medium with 10% FCS, 100 IU/ml penicillin and 100 μg/ml streptomycin. Nevirapine (NVP, Sigma) was used at a working concentration of 5 μM for the duration of the experiment, Aphidicolin (APH, Sigma) was added to cells at the concentration of 8 μM for 24 h before infection and maintained for the duration of the experiment.

*αCentauri sequences*

Amino acid sequences of NanoLuc, as set for in SEQ ID NO: 2 and SEQ ID NO: 3, or superfolder GFP as set for in SEQ ID NO: 5 and SEQ ID NO: 6 have been used.

*Construction and production of αCentauri viruses and vectors*

All viruses were HIV-1 LAI, either full-length or Δenv and pseudotyped with the vesicular stomatitis virus glycoprotein (VSV-G). $\alpha HIV^{GFP}$, $\alpha HIV\text{-}225T^{GFP}$ or $\alpha HIV^{Nluc}$ viral constructs were generated by polymerase chain reaction (PCR) using a pBlueScript (pBS) plasmid containing a PstI-NcoI fragment of the HIV-1 LAI wt or mutant 225T molecular clone. Briefly, oligonucleotides coding for αGFP or αNluc flanked by EcoRI and NdeI restriction sites were used to amplify and add αGFP or αNluc in C-ter of HIV-1 integrase.

The forward primer was 5'-CCAGTACTACGGTTAAGGC-3' (SEQ ID NO:7).

Reverse primers were αGFP 5'-GAAACATACATATGCTATGTAATCCCAGCAGCATTTACGTACTCATGAAGGACCA TGTGGTCACGAGCGGCCGCATCCTCATCCTGTCTACTTG-3' (SEQ ID NO:8) and αNluc 5'-GAAACATACATATGTTACGCCAGAATGCGTTCGCACAGCCGCCAGCCGGTCACT CCGTGGTCACGAGCGGCCGCATCCTCATCCTGTCTACTTG-3' (SEQ ID NO:9).

PCR products were digested with EcoRI/NdeI and cloned into pBS-LAI (PstI-NcoI). Finally, the PstI-NcoI fragment of LAI containing IN fused to αGFP or αNluc was cloned back into a wild-type Env or ΔEnv HIV-1 LAI molecular clone. αHIV-D116I$^{Nluc}$ was obtained by site-directed mutagenesis using the QuikChange II site-directed mutagenesis kit (Agilent) on the pBS-LAI containing IN fused to αNluc, and was then cloned back into a wild-type Env or ΔEnv HIV-1 LAI molecular clones.

Lentiviral vectors (LV) coding for CenNLS or CenNup214 were obtained by cloning HA-NLS or HA-Nup214 downstream of Cen$^{GFP}$ or Cen$^{Nluc}$ by PCR amplification. Cen$^{GFP}$NLS was generated by strand-overlap PCR first by generating Cen$^{GFP}$-HA from a GFP1-10 plasmid using the following primers 5'- GATCGGATCCCGCCACCATG (SEQ ID NO:10) and 5'-AAGAGCGTAATCTGGAACATCGTATGGGTAGCCGGCGCCTTTCTCGTTTGGGTCT TTGCTCAGC-3' (SEQ ID NO:11)

Then, GFP-HA-NLS was generated by a second PCR reaction and cloned into a HIV-1 derived vector with BamH1/XhoI restriction enzymes. Cen$^{NLuc}$NLS was synthesised by Genscript and cloned into a pcDNA3.1(+), followed by a HIV-1 derived vector. CenNup214 constructs were generated by amplifying Cen$^{GFP}$-HA or Cen$^{NLuc}$-HA with AgeI and NotI overhangs and subcloning these at the place of EGFP upstream of Nup214 using a pEGFP-Nup214 plasmid (Euroscarf).

All viruses and vectors were produced by transient transfection of HEK 293T cells by calcium phosphate precipitation with the proviral or LV plasmid, co-transfected with VSV-G expression plasmid for Δenv viruses and vectors, and with an encapsidation plasmid (pCMVΔR 8.74) for vectors. Viruses and vectors were harvested at 48 h after transfection. Viruses were concentrated using Lenti-X Concentrator (Clontech) and vectors by ultracentrifugation for 1 h at 64,000 × g (Beckman Coulter) at 4 °C.

*Centauri expression*

Cells were transduced with CenNLS$^{GFP}$ and CenNLS$^{NLuc}$ at MOI 10. Cells were used at 48 hours post-transduction (hpt), or stable cell lines were generated by selection and expansion of clones using Neomycin (1mg/ml). Cells were transfected CenNup214$^{GFP}$ and CenNup214$^{NLuc}$ plasmids using Fugene6 (Roche, HeLa) or calcium phosphate precipitation (HEK 293T) using 2 μg/ $10^6$ cells. The efficiency of transduction and transfection was assessed by indirect immunofluorescence labeling of the HA tag that was inserted in the corresponding Centauri construct followed by flow cytometry or confocal microscopy. A threshold of 80% HA+ cells was set as a minimum value for performing αCentauri experiments.

*Titration of αHIV stocks and infection*

Virus yields were measured by p24 ELISA according to the manufacturer's instructions (Clontech). Multiplicities of infection were estimated by assuming that 1 ng of p24 corresponds to 5,000 transducing units (TU) [4]. Viruses were treated with after benzonase (Sigma, 15min, 37°C). Unless otherwise indicated, cells were infected at 2.5 TU/cell. Alternatively, viruses were titered by measuring Pol copy numbers by quantitative PCR (qPCR) at 6 hours post-infection (hpi) in HeLa cells and infections were performed at given Pol copy numbers/cell. Unless otherwise stated, cells were infected at 2 Pol copies/cell.

*Analysis of αCentauri$^{GFP}$ complementation*

αCen$^{GFP}$ reconstitution was assessed at 48 hpi by flow cytometry on fixed cells. Alternatively, cells were seeded in 96-well glass-bottomed Sensoplates (Greiner) at 10.000 cells/well at 24 hpi, and acquired on a ThermoCellomics at 48 hpi after addition of live Hoechst 33342 (Molecular Probes).

*Analysis of αCentauri$^{NLuc}$ complementation*

CenNLS$^{NLuc}$ expressing cells, either transiently transduced or stable cell lines, were seeded in white opaque 96-well plates (Greiner) and infected the following day. αCen$^{Nuc}$ reconstitution was assessed at 24 hpi by adding NanoGlo substrate (Promega) according to the manufacturer's instructions. Other NLuc substrates were prepared by diluting the

stock solution 1:50 in assay buffer (100mM MES pH:6.0 adjusted with KOH, 1mM CDTA, 0.5% v/v Tergitol, 0.05% v/v antifoam, 150mM KCl, 1mM DTT et 35mM ThioUrea) and added to the cells 1:1. Luminescence was measured within 10 min using Tecan Infinite F200 Pro with 1000 ms integration and automatic attenuation.

*β-Galactosidase and Bradford Assays*

β-galactosidase assay was performed 48 hpi in indicator P4 cells according to the manufacturer's instructions (Roche Applied Science). Luciferase and β-Galactosidase activities were normalized for protein concentration by the Bradford assay. Luminescence and absorbance were acquired on a Tecan Infinite F200 Pro.

*Quantitative PCR*

Total cellular DNA was isolated at 24 hpi using the QIAamp DNA micro kit (Qiagen). Two long-terminal repeat (2-LTR)-containing circles were detected with primers MH535/536 and probe MH603 (Butler *et al.,* 2001), using as the standard curve the pUC-2LTR plasmid, which contains the HIV-1 2-LTR junction. Reactions were normalized by amplification of the late reverse transcript with primers MH531/532 and probe LRT-P (Butler et al., 2001) or quantification of HIV-1 POL gene using specific primers as published previously (Iglesias *et al.,* 2011). Alu-PCR was performed as published previously (Dinunzio *et al.,* 2012). Sequences of the different primers and probes correspond to SEQ ID NO:12 to SEQ ID NO:56;

*Antibodies and Stains*

The primary antibodies used were rat anti-HA tag (Roche 3F10), mouse monoclonal anti-p24 clone AG3.0 & 183-H12-5C (NIH AIDS Reagent Program). Secondary antibodies were goat anti-mouse and anti-rabbit HRP conjugates (Thermo Fisher Scientific, Rockford, IL) or Alexa Fluor 455 or 647 conjugates. Intracellular Gag was measured using KC57 antibody conjugated to FITC or PE (Beckman Coulter).

*Microscopy immunolabeling and imaging*

Cultures were rinsed with PBS and fixed with 4% paraformaldehyde (Electronic microscopy grade, Alfa Aesar) in PBS for 10 min at room temperature, treated with 50mM $NH_4Cl$ for 10min, permeabilized with 0.5% Triton X-100 for 15 min, and blocked with 0.3% BSA for 10 min. Cells were incubated with primary and secondary antibodies for 1 h and 30 min, respectively, in a moist chamber. Nuclei were labelled with Hoechst dye (Molecular Probes). Images were acquired using a LSM700 (Zeiss) confocal microscope equipped with a 63x objective, or by Airyscan LSM800 (Zeiss) both operated by the Zen software. Image analysis was performed using ImageJ.

*Quality controls*

Z' factors were calculated using 10 to 30 replicates per condition, randomly distributed on the plate. The Z-factor (Zhang *et al.,* 1999) is a measure that quantifies the separation between the distribution of positive and negative controls.

$$\mathrm{Z-factor} = 1 - \frac{3(\sigma_p + \sigma_n)}{|\mu_p - \mu_n|}$$

where $\mu_p$ and $\sigma_p$ are the mean and standard deviation values of the positive control and $\mu_n$ and $\sigma_n$ are those of the negative control.

If robust, the Z-factor is calculated using robust estimates of location (median) and spread (mad).

The signal/background ($\mu_p$ - $\mu_n$) and signal/noise (($\mu_p$ - $\mu_n$)/ $\sigma_n$) ratios are also provided.

All statistical analysis was performed with R programming language.

*Generation of luciferin solutions from the O-acetylated luciferins*

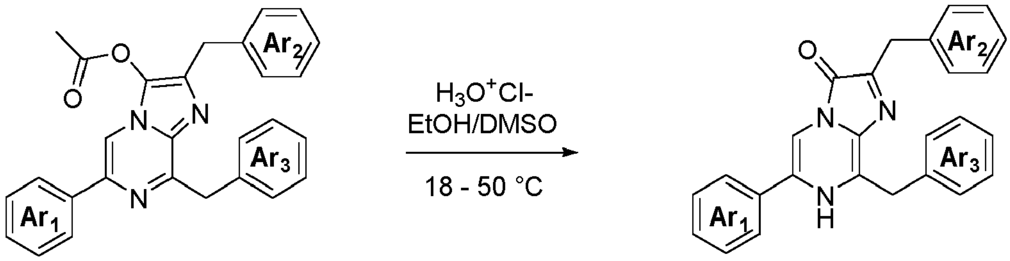


The considered O-acetylated luciferin (1 mg) was dissolved in DMSO (0.2 mL) and then diluted by adding a solution of acidic ethanol (0.3 ml) made from the addition of 37 % hydrochloric acid (100 μl) on 100 % ethanol (12 mL). The 0.5 mL reaction solution was incubated at 50°C for 2 h to give a stock solution which was aliquoted and frozen at -80°C for later use. The following O-acetylated luciferins (hikarazines) were used in this work:

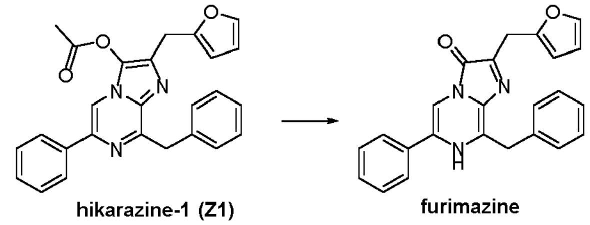

**hikarazine-1 (Z1)** **furimazine**

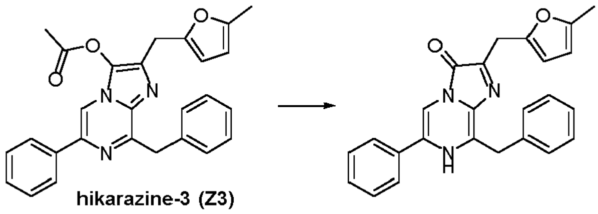

**hikarazine-3 (Z3)**

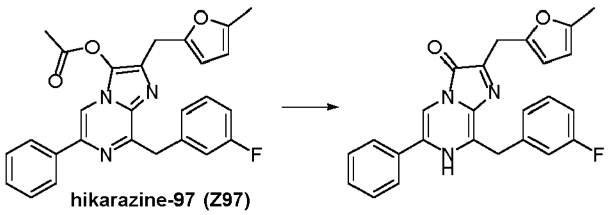

**hikarazine-97 (Z97)**

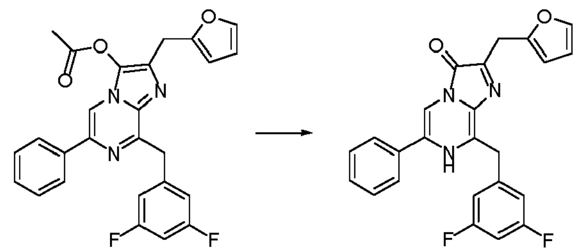

**hikarazine-103 (Z103)**

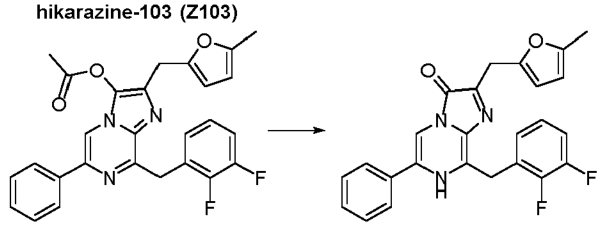

**hikarazine-108 (Z108)**

*RNA interference*

Lentiviral vectors (LV) coding for shRNA against Pin1, CypA, RanBP2, TNPO1, TNPO3, CKAP1, WIRE, MAP1A, MAP1S, IPO5, IPO7 and KPNB1 were generated as previously published (Di Nunzio *et al.,* 2012 ; Fernandez *et al.,* 2015 ; Fernandez *et al.,* 2019 ; Maarifi *et al.,* 2019 ; Kaul *et al.,* 2009). Transduction was performed at MOI 50.

*Statistical analyses*

Unpaired and paired t tests, ordinary One-Way ANOVA, and $R^2$ coefficients were obtained using Prism 6.

Results

The HIV particle comprises an envelope, a capsid, and two copies of positive-strand RNA genome. Following the fusion of the HIV envelope with the target cell membrane, the capsid is released into the cytoplasm and transported towards the nucleus. Reverse transcription of the genome into double-stranded DNA produces a pre-integration complex (PIC), which enters the nucleus by active transport through the nuclear pore complex (NPC) and mediates integration of the HIV DNA into the host cell chromatin.

In the assay, the αCen reporter is expressed as two complementary, self-assembling fragments of sfGFP or NLuc. The small α fragment ($\alpha^{GFP}$: 16aa, or $\alpha^{NLuc}$: 13aa) was fused into full-length and Δenv HIV-1 molecular clones (hereafter αHIV) in C-ter of HIV-1 integrase (IN) (**Fig. 1c**).

Each incoming viral particle contains approximately 120 IN molecules bound to the RNA genome, based on the 20:1 synthesis ratio of Gag to Gag-Pol. After reverse transcription and shedding of the capsid, IN molecules that bind to the viral DNA ends as a multimer accompany the PIC into the nucleus, while free cytoplasmic IN is inherently unstable and likely undergoes proteasomal degradation.

Insertion of the α-tag within the Pol coding sequence did not disrupt particle production **(Fig.1d)** and ensured wild-type viral infectivity compared with non-tagged viruses (**Fig. 1e**).

In parallel, the large fragment ($Cen^{GFP}$ 52 kDa, or $Cen^{Nluc}$ 39 kDa) was fused to the nucleoporin Nup214 for targeting to the NPC (CenNup214) or to the triple nuclear localization signal (NLS) of SV40 for tethering to the nucleus (CenNLS) (**Fig. 1f**). A HA tag was introduced in all Cen constructs to monitor their expression and localization (**Fig. 1g).**

To assess αCentauri protein complementation following viral trafficking to the nuclear envelope or to the nucleus, HeLa cells expressing $CenNup214^{GFP}$ or $CenNLS^{GFP}$ were infected with $\alpha HIV^{GFP}$. Transduced cells did not emit any GFP fluorescence upon Centauri expression alone, however infection by αHIV led to gain-of-GFP signal close to nuclear pores in CenNup214 cells (**Fig. 2a**), and in nuclei in CenNLS cells (with 87% of all GFP signal localizing to the nucleus) (**Fig. 2b**).

To further characterize αCentauri protein complementation following virus entry into the nucleus, several cell lines including T cells, which are the relevant target cells of HIV *in vivo,* were transduced with CenNLS and infected with αHIV. Complementation of sfGFP led to an approximately 5-10-fold increase in fluorescence in all tested cell types, while neither the α nor the Cen fragments emitted any detectable signal when expressed alone (**Fig. 2c**).

To exclude that complementation occurred upon mixing of cytoplasmic and nuclear contents during cell division, Aphidicolin (APH) was used as control to block nuclear envelope breakdown during mitosis. Treatment did not reduce signal, confirming that αCentauri signal is generated following viral transport through NPCs, which is concordant with the HIV PIC entering the nucleus through nuclear pores (**Fig. 2d**).

To assess whether free IN not associated with the PIC might enter the nucleus and generate signal, we performed infections in the presence of Nevirapine (NVP), a reverse transcription inhibitor that blocks PIC formation and thus prevents viral ribonucleoprotein entry into the nucleus. Treatment of cells with NVP entirely abolished αCentauri complementation, suggesting that free IN molecules do not enter the nucleus of infected cells (**Fig. 2d**).

In addition, nuclear PCA was inhibited following infection with a HIV-1 mutant that is defective for nuclear entry (225T - Zennou *et al.,* 2000), confirming that viral nuclear import is both necessary and sufficient for CenNLS complementation (**Fig. 2e**). Nevertheless, the use of sfGFP as a reporter came with some limitations. First, although signal was detectable as of 24 hpi, complementation was optimal at 48 hpi (**Fig. 2f**), which comes substantially later than HIV-1 nuclear import estimated to occur 8-12 hpi in non-synchronised infections.

Second, indirect immunofluorescence detection of Gag indicated that only ~60% productively infected cells were αCentauri-positive (**Fig. 2g**). These limitations underline the lower sensitivity of the fluorescent reporter and likely reflects the need for it to assemble and accumulate to reach a detectable threshold.

We therefore adapted αCentauri to a luminescence reporter, since this reporter system exhibits a wide dynamic range with a high sensitivity and virtually no background. Complementation of NLuc following infection of $CenNLS^{NLuc}$ cells with $\alpha HIV^{NLuc}$ was measured by plate luminometry.

Infection resulted in 5- to 200-fold increase in emitted luminescence signal in all tested cell types, including T cells (**Fig. 3a**). The luminescence signal was proportional to CenNLS expression level as illustrated in HeLa cells (**Fig. 3b**). Signal was detected as early as 16 hpi and was optimal at 24 hpi (**Fig. 3c**), and was also detectable following infection with wild-type envelope HIV (**Fig. 3d**).

Moreover, the αCentauri signal intensity was directly proportional to the detection of the HIV-1 genome in the nucleus, confirming that the NLuc reporter was reconstituted upon HIV-1 PIC nuclear import (**Fig. 3e**).

Since αCentauri measurements were performed several hours after viral nuclear import, it was likely impossible that PCA also occurred following nuclear entry of neo-synthesised α-tagged IN.

HIV-1 Gag and Gag-Pol polyproteins have been shown to traffic back to the nucleus and perinuclear area after translation in the cytoplasm. We therefore introduced a D116I mutation in viruses to block integration and all downstream steps of viral replication. Infection with D116I virus still achieved ca. 10-fold increase in NLuc compared to infection in the presence of Nevirapine (**Fig. 3f**), confirming that αCentauri allows the detection of viral PICs trafficking into the nucleus.

In principle, the *α*CentauriNLS assay reflects the combined efficiency of all the early steps of viral replication required to reach the nucleus, and is not specific to nuclear import. However, we reasoned that by performing αCentauriNup214 and *α*CentauriNLS side-by-side in the context of a single high screening platform, the system would allow to deconvolute successful trafficking to the NPC from HIV nuclear import and therefore enable the specific screening of nuclear import. Since the sfGFP approach was poorly quantitative for Nup214, we tested the NLuc readout. To assess αCentauri protein complementation following virus docking at the nuclear envelope, HeLa cells were transduced or transfected with CenNup214, then infected with αHIV. Infection resulted in a 10-fold increase in signal, indicating that the *α*CentauriNup214 assay is quantitative and could be used in parallel to *α*CentauriNLS to screen for specific inhibitors of nuclear import (**Fig. 3g**).

Currently, the paramount screening strategy for HIV replication relies on the HIV long-terminal repeat promoter (LTR) whose transcriptional activity is turned on by the early HIV gene product, Tat. LTR-reporter cell lines, using LacZ, eGFP or Luciferase, have been used in the past to identify cellular co-factors of HIV infection. Alternatively, infected cells can be scored by immunolabelling of viral antigens. We have therefore compared αCentauri$^{GFP}$ and αCentauri$^{NLuc}$ assays with an LTR-LacZ system and with the labelling of intracellular Gag (iGag) to score HIV- infected cells. Results revealed that the αCentauri$^{NLuc}$ system exhibits a wide dynamic range with a max/min ratio of 65, compared with 19 for the LTR-LacZ system and <3 for αCentauri$^{GFP}$ and iGag (**Fig. 4a**). The assay also exhibited high sensitivity, virtually no background, and a linear dose-response over 2-log, which was superior to any other tested assay (**Fig. 4a**).

This assay initially used the commercially available Nano-Glo bioluminescence-based reporting system which is made of the NanoLuc/NanoKAZ luciferase and uses furimazine as its substrate. In an attempt to improve its sensitivity, we also evaluated a series of coelenterazine analogues including furimazine (Z01) that were previously characterized as NanoLuc substrates. Accordingly, the corresponding O-acetylated proluciferins (hikarazines 01, 03, 97, 103 and 108) were hydrolysed and the resulting solutions of these luciferin analogues assessed at a final concentration of 40-50 μM.

As compared with the Nanoglo kit, which was used according to manufacturer's instructions, all these luciferin analogues actually led to a greater signal intensity. Even hikarazine-1 (Z01), which gives furimazine, provided a slightly improved bioluminescence signal. Particularly noteworthy are the luciferin solutions obtained from Z03, Z97 and Z108 which gave the highest RLU values following infection with αHIV$^{NLuc}$, with respectively signals 6-, 11- and 11-fold higher than the ones observed with Nano-Glo (**Fig. 4b**).

The best discrimination between positive and negative samples was obtained with Z03 (30-fold), Nano-Glo (20-fold), Z97 (17-fold) and Z01 (10-fold) (**Fig. 4b**). The lowest ratio between αHIV$^{NLuc}$ over αHIV$^{NLuc}$ + NVP was obtained with Z103 (5-fold). Therefore, we suggest that the *O*-acetylated coelenterazine analogues hikarazine-03 (Z03) and hikarazine-97 (Z97), which lead to the corresponding luciferins upon hydrolysis, are particularly suited for performing αCentauri$^{NLuc}$ screens.

In order to validate our assay for screening, we also assessed plate uniformity and min-to-max discrimination by performing assays in 96-well format. Plates were laid out in interleaved format by alternating positive and negative controls. This enabled us to calculate the Z' factor, an indicator of the quality of a screening assay. Both αCentauri$^{GFP}$ and αCentauri$^{NLuc}$ presented good discrimination between positive and negative controls with Z' of 0.63 for αHIV$^{GFP}$, 0.74 for αHIV$^{NLuc}$ and 0.35 for αHIV-D1161$^{NLuc}$ compared with the Nevirapine negative control (**Fig. 4c**).

Signal/background ratios were 2.53 for αHIV$^{GFP}$, and 30.26 and 5.07 for αHIV$^{NLuc}$ and αHIV-D116I$^{NLuc}$, respectively, which was considered acceptable since >2. Signal/noise ratios were 34.32 for αHIV$^{GFP}$, and 280.77 and 39.07 for αHIV$^{NLuc}$ and αHIV-D116I$^{NLuc}$, respectively, which was considered acceptable since >10.

Finally, to achieve a proof-of-concept, we performed a small shRNA-based screen that included proteins known to mediate HIV-1 nuclear import, as well as related proteins previously shown to have little effect on HIV-1. Among proteins known to promote HIV entry in the nucleus, MAPIA and MAP1S contribute to retrograde trafficking, RanBP2 anchors capsids at the NPC, TRN-1/TNPO1 triggers productive uncoating, TNPO3 indirectly regulates capsid stability via its cargoes CPSF6. Among proteins that were not expected to have much effect on entry of the viral genome in the nucleus, CypA has no effect in HeLa cells, KPNB1 mediates Tat nuclear import independently of PIC entry, □-karyopherins IPO5 and IPO7, and cytoskeletal proteins CKAP1 and WIRE have a low to moderate effect on infection. Hela-CenNLS$^{NLuc}$ cells were treated with previously validated shRNAs against these cellular co-factors, infected with αHIV$^{NLuc}$, and NLuc signal was measured at 24 hpi. The knockdown of RanBP2 and TRN-1/TNPO1 had the greatest effect on HIV nuclear import, leading to ~10-fold decrease in αCentauri. Other proteins that came out as relevant co-factors were Pin1, MAPIA and MAP1S, thus confirming previous findings. Conversely, shRNAs against CKAP1, TNPO3, IPO5, IPO7 and KPNB1 led to marginal reduction in αCentauri signal (**Fig 4d**).

Results confirm that αCentauri is a quantitative and reliable assay, applicable to the screening of shRNA/CRISPR-Cas9 libraries or small compound libraries.

## Innate immunity

### Material & Methods

*Cells and drugs*

P4 TAR-β-gal indicator cells are HeLa CD4+ CXCR4+ CCR5+ carrying the LacZ gene under the control of the HIV-1 LTR promoter (AIDS Reagent Program). HEK 293T (CRL-11268), Hela (CCL-2), HT1080 (CCL-121) and A549 (CCL-185) were obtained from the ATCC. The MT4R5 (Amara et al., J.Virol., 2003) and CEM CD4+ (AIDS Reagent Program) T cell lines were grown in RPMI medium with 10% FCS, 100 IU/ml penicillin and 100 μg/ml streptomycin. Vero E6 and HCT-116 are both from the ATCC repository.

Puromycin, G418 and Hygromycin were obtained from Sigma and used at different concentration for cell selection.

*αCentauri sequences*

Amino acid sequences of NanoLuc, indicating the site of the split as a vertical line, are as follows: NanoLuc (SEQ ID NO:1) (based on Dixon et al., ACS Chem. Biol., 2016):

*Construction of αCentauri plasmids and vectors*

CenNLS and IRF3-, IRF7- and p65-α-Flag expressing vectors were synthesised by GenScript into a pcDNA3.1(+) vector using NotI/XhoI cloning site. These constructions were also cloned into HIV-1 derived vectors using BamHI/XhoI cloning site.

Vectors were produced by transient transfection of HEK 293T cells by calcium phosphate precipitation with LV plasmid, co-transfected with VSV-G expression plasmid and with an encapsidation plasmid (pCMVΔR 8.74). Vectors were harvested at 48 h after transfection and concentrated by ultracentrifugation for 1 h at 64,000 × g (Beckman Coulter) at 4 °C.

*Analysis of αCentauri$^{NLuc}$ complementation*

CenNLS expressing cells, either transiently by plasmid transfection or HIV-1 derived vector transduction, or stably by selection under antibiotic, were seeded in 50μl of complete growth medium in white opaque 96-well plates (Greiner) and stimulated the following day with defective interfering Sendai Virus (SdV, provided by D. Garcin (Department of Microbiology and Molecular Medicine, University of Geneva, Geneva, Switzerland) and used at 50 hemagglutination units (HAU)/ml , for 2 to 6 hours. αCen$^{Nuc}$ reconstitution was assessed by adding NanoGlo substrate (Promega), which is fumirazine, according to the manufacturer's instructions, or other coelenterazine analogues including furimazine (Coutant et al., 2019, 2020), which were prepared by diluting the stock solution 1:50 in assay buffer (100mM MES pH:6.0 adjusted with KOH, 1mM CDTA, 0.5% v/v Tergitol, 0.05% v/v antifoam, 150mM KCl, 1mM DTT et 35mM ThioUrea) and added to the cells 1:1. Luminescence was measured within 10 min using Tecan Infinite F200 Pro with 1000 ms integration and automatic attenuation.

*Antibodies and Stains*

Expression of α and Cen was assessed by immunolabelling of HA or Flag tag and analysed by confocal microscopy, flow cytometry or western-blot. The primary antibodies used were rat anti-HA tag (Roche 3F10) and mouse monoclonal anti-flag clone M2 (Sigma). Secondary antibodies were goat anti-mouse and anti-rat HRP conjugates (Thermo Fisher Scientific, Rockford, IL) or Alexa Fluor 488 or 647 conjugates.

*Generation of luciferin solutions from the O-acetylated luciferins*

The luciferin solution was prepared as described above for "Viral trafficking".

**Results**

The present invention proposes innovative tools to characterise and quantify by HTS the nuclear translocation of transcription factors implicated in innate immune signalling pathways. This technique is based on the complementation of protein fragments to reconstitute a functional protein, an approach known as Protein-fragment complementation assay (PCA), which has been adapted to the measure of a nuclear translocation event. NanoLuc (Nluc) is an engineered luciferase derived from a deep sea luminous shrimp that has a mass of 19kD, making it much smaller than *Renilla* (36kD) or firefly (61kD) luciferases, and therefore more appealing for fusion protein construction. Moreover, Nluc is also approximately 150x brighter than other commercially available luciferase reporters allowing very sensitive detection (Hall, M.P. et al. ACS Chem Biol 7, 1848-1857, (2012)).

The innovative nature of this technology lies on the PCA of NLuc exclusively upon the nuclear translocation of transcription factors tagged to the C-terminal 13-residue fragment of Nluc, which is hereby called fragment α. Specifically, tagging transcription factors such as IRF3, IRF7 and the p65 subunit of NF-κB have already been tested. These encounter the large complementary Nluc fragments (fragment Cen) stably expressed and sequestered in the nucleus due to their fusion with a tripartite nuclear localisation signal (NLS) (**Fig. 5**). Neither of the two fragments can emit luminescence alone, but translocation of the transcription factors to the nucleus will result in the reconstitution of functional Nluc proteins. The complementation of Nluc occurs via the self-association of the α and Cen fragments in the confined environment

of the nucleus, and leads to a bioluminescent signal that is readily measured by plate luminometry. The assay has been termed αCentauri to underline its binary and very bright nature that is reminiscent of the αCentauri star system.

The αCentauri tools were originally developed for HTS to screen compounds by quantitatively measuring their impact on innate immune or inflammatory response signalling pathways, independently of the transcriptional and translational machineries. However, since all viral infections trigger signalling pathways downstream of PRR to varying degrees, which all converge on the phosphorylation and nuclear translocation of IRF3, the system of the present invention should provide a fast and reliable read-out for any viral experimental infection.

The proof-of-concept of the approach was performed on HEK-293T and HeLa cells infected with Sendai Virus (SeV), a RNA virus that causes severe respiratory disease in mice. HeLa cells were transduced with a lentiviral vector expressing Cen-NLS, and cellular clones were generated. Next, these clones were transiently transfected with an IRF3α expression plasmid. HEK-293T cells were doubly transfected with plasmids expressing both Cen-NLS and IRF3α (**Fig. 6a**). αCentauri complementation was found to be 3-6 fold above uninfected controls (**Fig. 6b, c**). In the case of HeLa cell clones, we made certain that transduction of the lentiviral vector does not induce the synthesis of IFN-β in cells, unlike those infected with SeV. IFN-β is a cytokine secreted by cells downstream of the signaling cascade leading to nuclear translocation of the transcription factor IRF3. Thus, results show that the nuclear translocation of IRF3α is only due to the infection of cells with SeV (**Fig. 6d**). Expression of the α and Centauri fragments was monitored by flow cytometry (data not shown) and confocal microscopy (**Fig. 6e**) upon detection of the Flag and HA tags that were inserted in the corresponding expression plasmids. In the absence of viral infection, both fragments were kept confined in separate cellular compartments: IRF3α in the cytoplasm and Cen-NLS in the nucleus. Strikingly, infection of these cells with SeV resulted in the nuclear relocalisation of IRF3α, and emission of NLuc luminescence signal (**Fig. 6f**) by bioluminescence microscopy. Similar results have been obtained in other cell lines that were tested to this day (HCT116, HT1080, A549).

Several cell-based assays are commercially available to investigate the activity of transcription factors under physiological conditions, but all are based on the measurement of the transcriptional activity (using ISRE-reporter systems). While some research groups already use these systems to screen molecules, there is a general consensus that their sensitivity and specificity are not high enough. In particular, compounds acting on transcription, translation, cell cycle, or metabolism will yield false positive response with these assays. The αCentauri technology offers a radical break from commercial approaches since it is based on protein complementation to monitor the nuclear translocation of transcription factors, rather than their transcriptional activity.

The approach of the present invention has been compared side-by-side with alternatives commercialised by Invivogen (ISRE-Luciferase and ISRE-Secreted Embryonic Alkaline Phosphatase/SEAP cell lines). Since the αCentauri system does not rely on cellular transcription and translation machineries, it may be used at very early time points, 6 hpi by SeV compared to 24 hpi for the ISRE systems (**Fig. 7a**). In addition, the αCentauri system appeared to be more specific (**Fig. 7b**) since it didn't react to false positives such as Histone Deacetylase (HDAC) inhibitors, known to unspecifically activate gene expression, and more sensitive since it responded to compounds to which other systems were unresponsive (**Fig. 7c**). Finally, αCentauri offers a simpler approach with fewer steps, thus favouring automation and fast HTS protocols.

Therefore, the approach of the present invention provides a robust, versatile and sensitive read-out of innate signalling activation following viral infection. It is more reliable than competing commercial assays that monitor the activity of transcription factor-responsive promoter elements, which are hampered by a high number of false positives and negatives linked to the effect of transcriptional modulators and inducers of genotoxic stress, such as intercalating agents, or molecules interfering with cell cycle.

## Claims

**1.** Immortal cell line that expresses a first fragment of a reporter protein in the nucleus of the cell and a modified transcription factor comprising a transcription factor fused to a second fragment of the reporter protein, said second fragment being complementary to the first fragment, wherein the modified transcription factor is able to mediate the innate immune response in the cells.

**2.** The immortal cell line of claim 1, wherein the first fragment of the reporter protein is fused to a protein or motif conferring localization to the nucleus, such as SV40, or to a protein of the nuclear pore complex, such as a nucleoporin, preferably Nup214, Nup98 or Nup153.

**3.** The immortal cell line of claim 1 or 2, wherein the first fragment of the reporter protein is fused to the protein of the nucleus via a flexible linker sequence.

**4.** The immortal cell line of any one of claims 1 to 3, wherein the second fragment of the reporter protein is fused to an interferon regulatory factor (IRF), a STAT protein or a subunit of NF-κB.

**5.** The immortal cell line of any one of claims 1 to 4, wherein the reporter protein is selected from the group consisting of a fluorescent protein, preferably a GFP-like fluorescent protein, and a bioluminescent protein, preferably a NanoLuc-like protein.

**6.** The immortal cell line of any one of claims 1 to 4, wherein the first fragment is α-NanoLuc with the amino acid sequence set forth in SEQ ID NO: 2 and the second fragment is Cen-NanoLuc with the amino acid sequence set forth in SEQ ID NO: 3.

**7.** Process for monitoring activation of innate immunity signaling pathways in a population of cells comprising:

- Providing cells according to any one of claims 1 to 6;
- Subjecting said cells to a stimulus suitable to activate innate immunity signaling pathways, and
- Detecting the reconstituted reporter protein, subsequent to translocation of the transcription factor of interest in the nucleus;

wherein detection of the reconstituted reporter protein in the cells is indicative of the activation of the innate immunity signaling pathways in the cells.

**8.** The process of claim 7 for use for screening candidate molecules able to stimulate and/or modulate innate immunity, wherein the stimulus consists in the candidate molecule(s) to be tested, wherein the detection of the reconstituted reporter protein in the cells is indicative that the candidate molecule is able to stimulate and/or to modulate the innate immunity.

**9.** The process of claim 7 for use for screening candidate molecules able to overstimulate innate immunity, wherein cells are submitted to a stimulus suitable to activate the innate immunity and to the candidate molecule(s) to be tested (before, during or after submitting the cells to the stimulus activating the innate immunity), wherein an increase of detection of the reconstituted reporter protein in the cells compared to reconstituted reporter protein in control cells, is indicative that the candidate molecule is able to over-activate the innate immunity.

**9.** The process of claim 7 for use for screening candidate molecules able to inhibit innate immunity, wherein the cells are submitted to a stimulus suitable to activate the innate immunity and to the candidate molecule(s) to be tested (before, during or after submitting the cells to the stimulus activating the innate immunity), wherein an absence of detection of the reconstituted reporter protein in the cells, or a decrease of detection of the reconstituted reporter protein in the cells compared to reconstituted reporter protein in control cells, is indicative that the candidate molecule is able to inhibit stimulation of the innate immunity.

**10.** A kit for screening candidate molecules acting on innate immunity pathway, said kit comprising at least one immortal cell line according to any one of claims 1 to 6, and optionally one or more agonist(s) of interferon and inflammation signaling adapted to the cell line provided in the kit and, optionally, a substrate for the protein reporter (e.g. fumirazine).

**11.** Process for monitoring the viral translocation of a virus of interest to a subcellular component of interest in a population of cells comprising:

- Providing cells that express a first fragment of a reporter protein tethered to the subcellular component of interest;
- Providing a virus of interest, wherein at least one viral protein has been tagged with second fragment of the reporter protein, said second fragment being complementary to the first fragment,
- Subjecting said cells to said virus;
- Detecting the reconstituted reporter protein in the cell of interest;

wherein detection of reconstituted reporter protein in said subcellular component or in the cell is indicative of the viral translocation of the viral protein to said subcellular component.

**12.** The Process of claim 11 for screening antiviral candidate molecules, wherein the cells are subjected both to the virus and to at least one antiviral candidate molecule, wherein an absence of detection of the reconstituted reporter protein in said subcellular component of the cells or in the cell or a decrease of detection of the reconstituted reporter protein in said subcellular component of the cells or in the cell compared to reconstituted reporter protein in control cells, is indicative that the candidate molecule is able to inhibit the viral infection.

**13.** Use of a split reporter protein for monitoring and/or evaluating and/or quantifying the viral trafficking of a virus of interest between subcellular compartments in a cell population, wherein a first fragment of the reporter protein is fused to a protein of a subcellular compartment of interest in the cell population, and the second fragment of the reporter protein is fused to a viral protein of the virus of interest.

**14.** The process of any one of claims 11 to 12 or use of claim 13, wherein the reporter protein is selected from the group consisting of a fluorescent protein, preferably a GFP-like fluorescent protein, and a bioluminescent protein, preferably a NanoLuc-like protein.

**15.** The process or use of any one of claims 11 to 14, wherein the first fragment is $\alpha$-NanoLuc with the amino acid sequence set forth in SEQ ID NO: 2 and the second fragment is Cen-NanoLuc with the amino acid sequence set forth in SEQ ID NO: 3.

**16.** The process or use of any one of claims 11 to 15, wherein the first fragment of the reporter protein is fused to a protein or motif conferring localization to a subcellular compartment selected from the group consisting of the nucleus, the endoplasmic reticulum, the nuclear pore complex or the mitochondria and/or wherein the second fragment of the reporter protein is fused to an integrase protein of the virus.

**17.** Kit comprising:

- an immortal cell line that expresses a first fragment of a reporter protein in a subcellular compartment of interest, and
- a virus wherein at least one viral protein has been tagged with a second fragment of the reporter protein, said second fragment being complementary to the first fragment; and
- optionally, a substrate for the protein reporter (e.g. fumirazine).

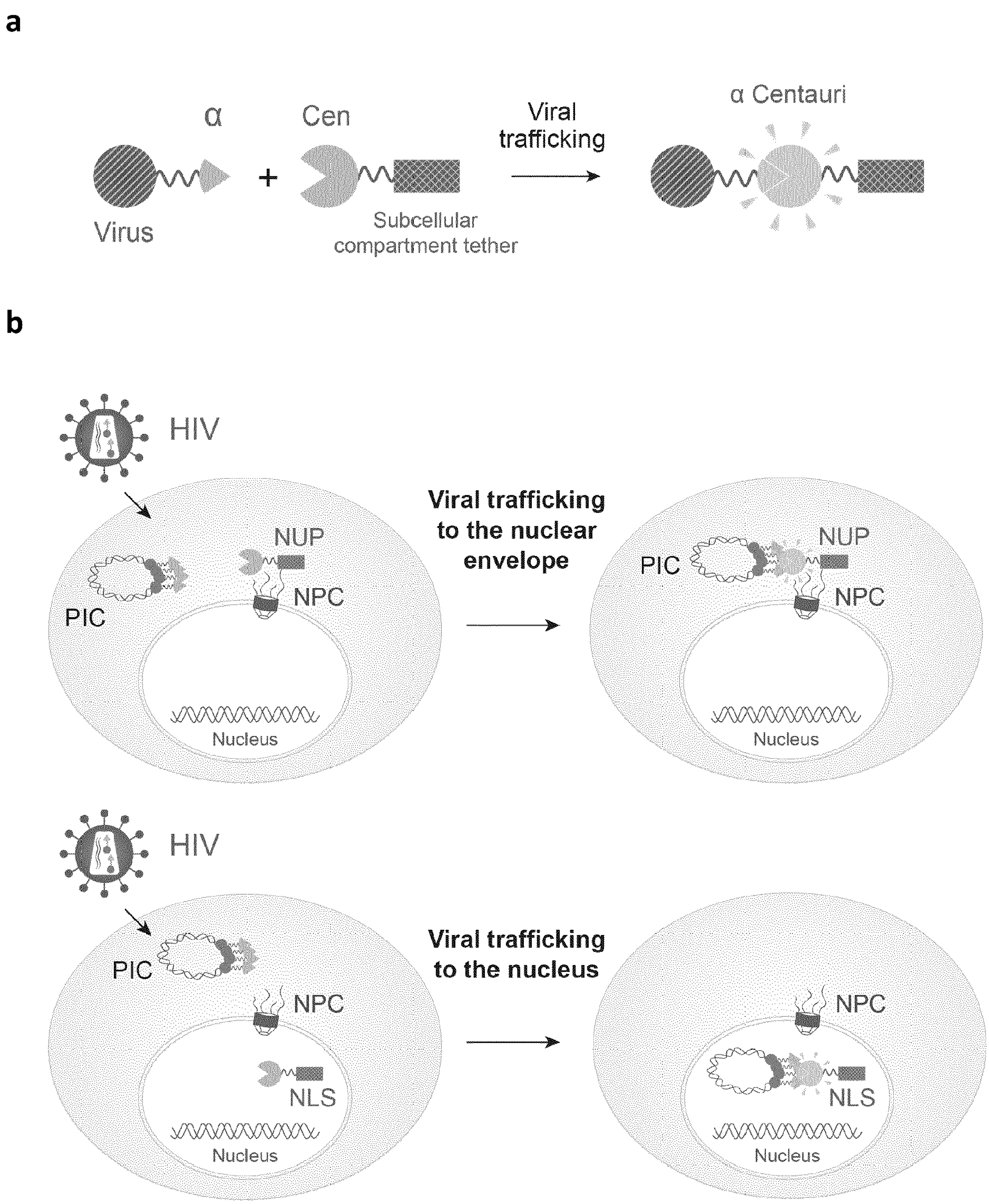


Figure 1 (continue)

c

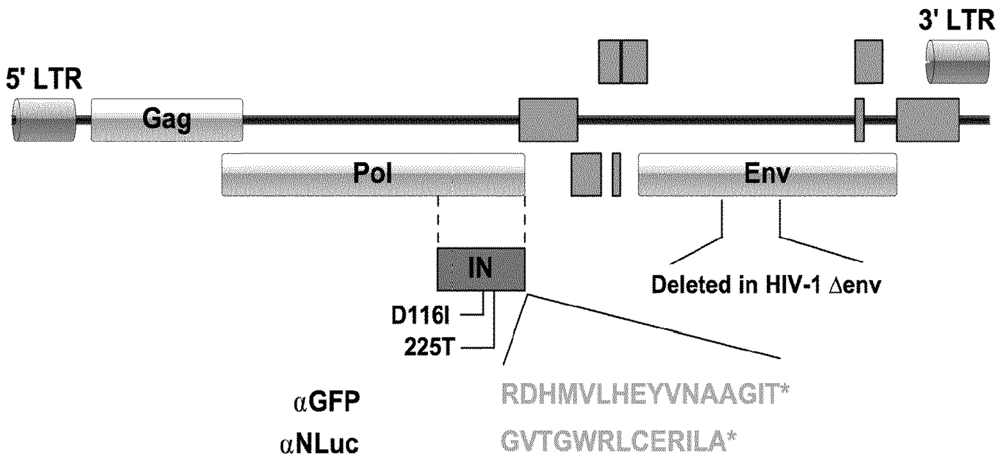


d

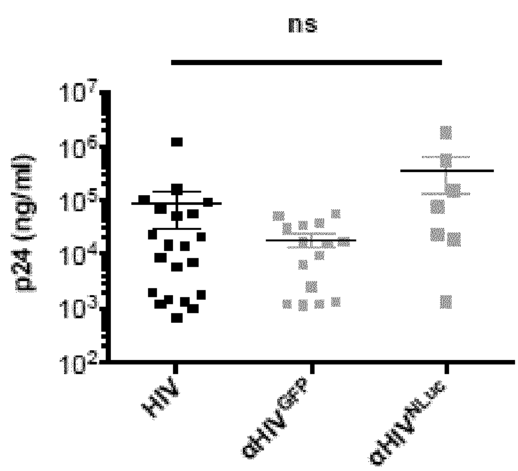


E

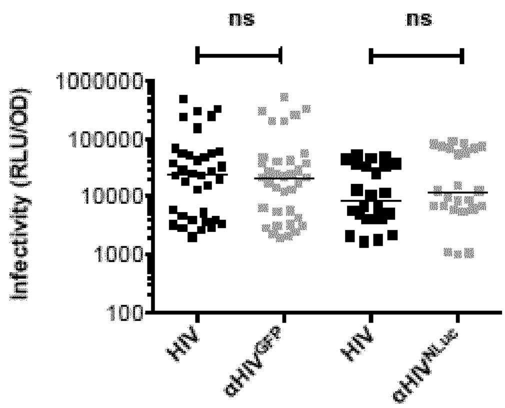


Figure 1 (continue)

f

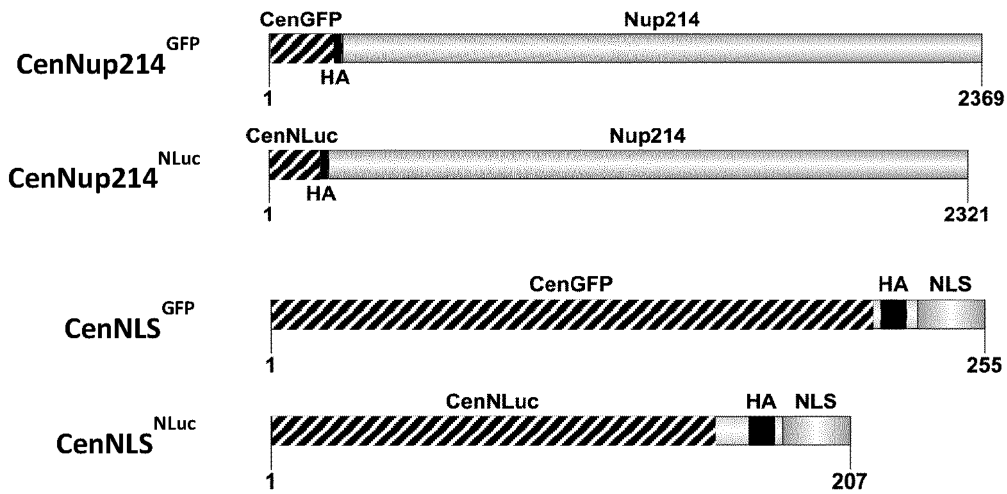


g

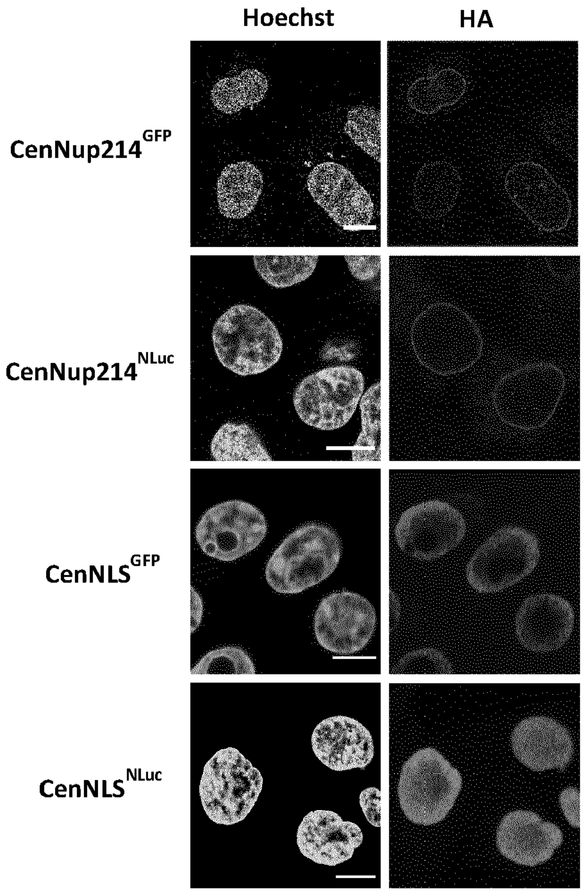


Figure 1 (final)

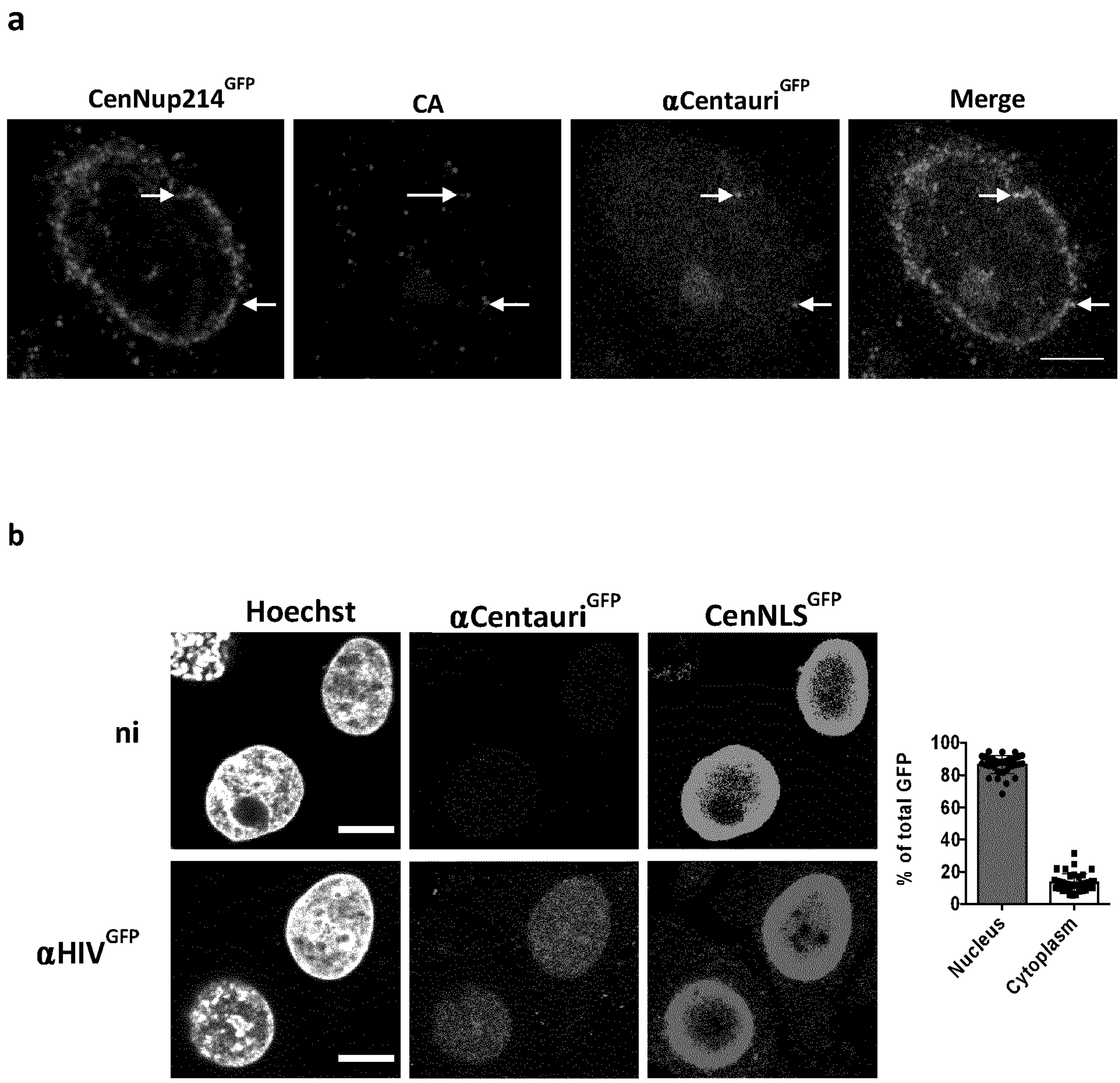


Figure 2 (continue)

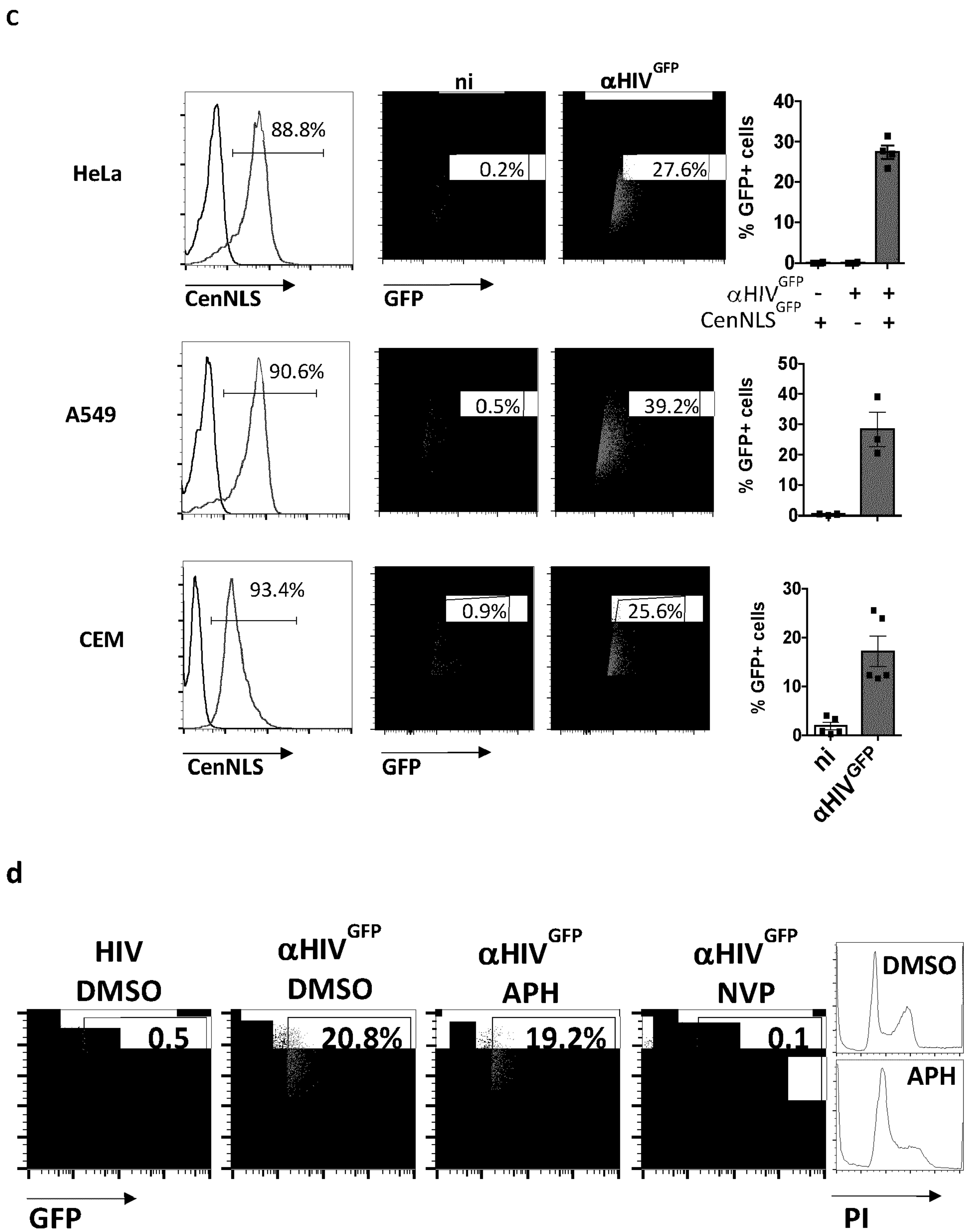


Figure 2 (continue)

e

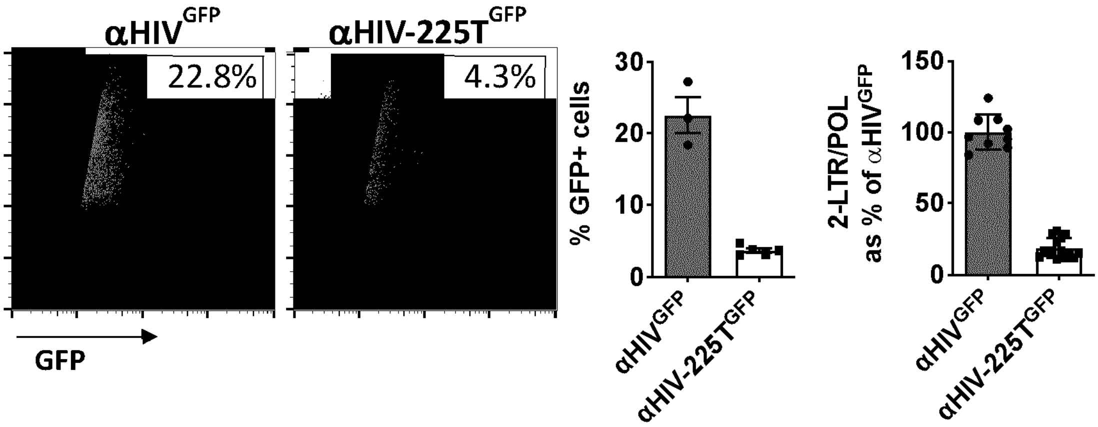


f

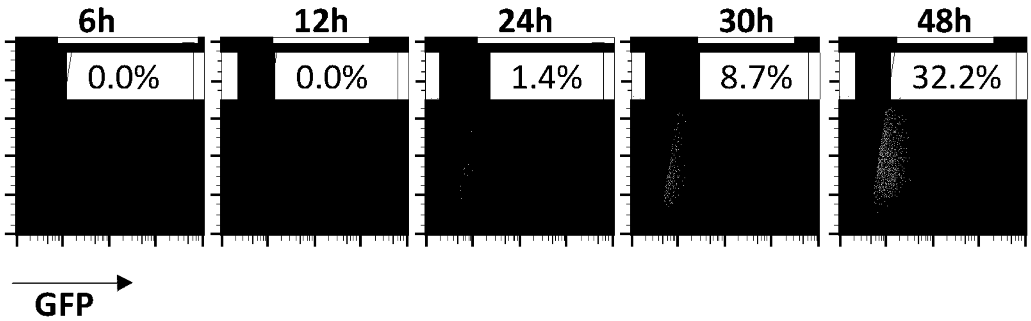


g

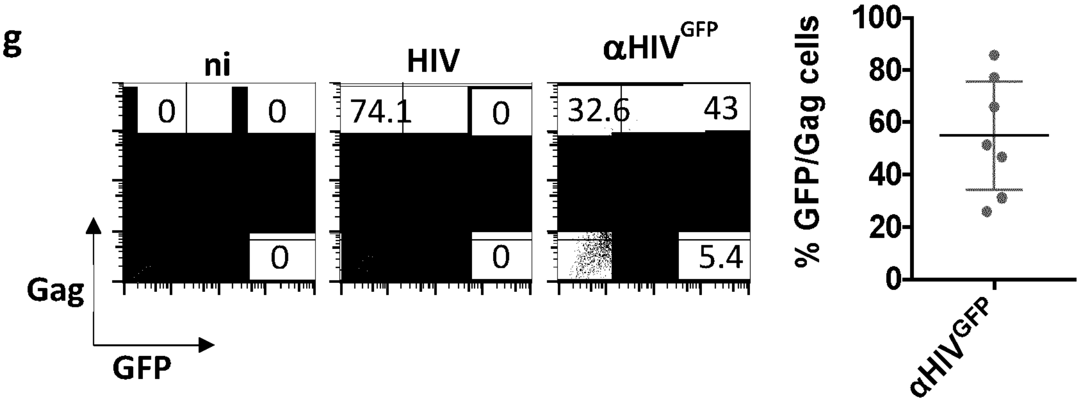


Figure 2 (final)

a

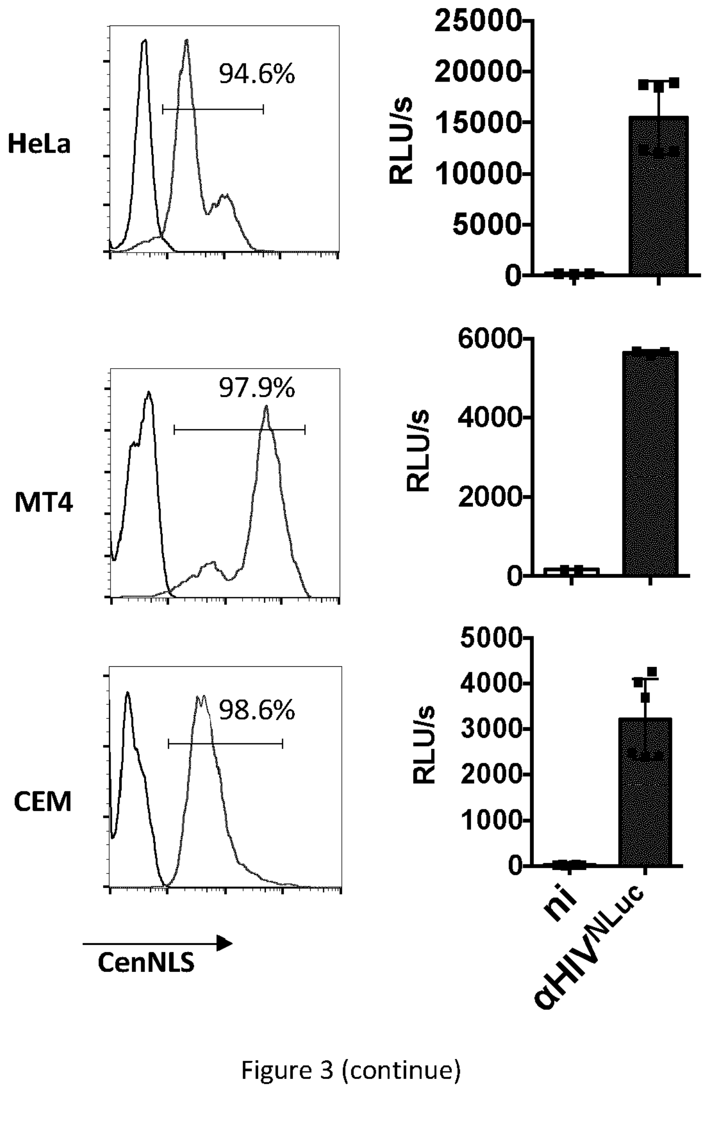


Figure 3 (continue)

b

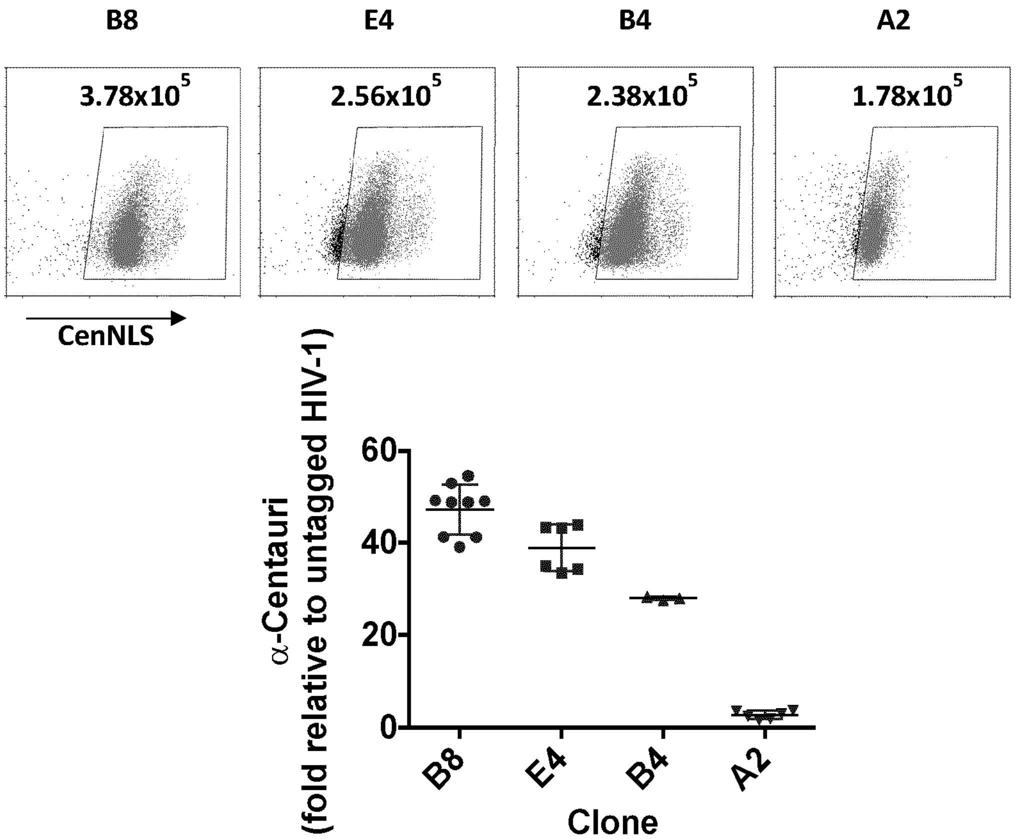


c

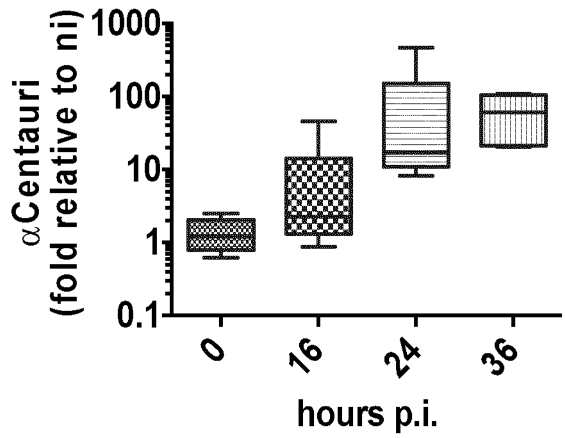


Figure 3 (continue)

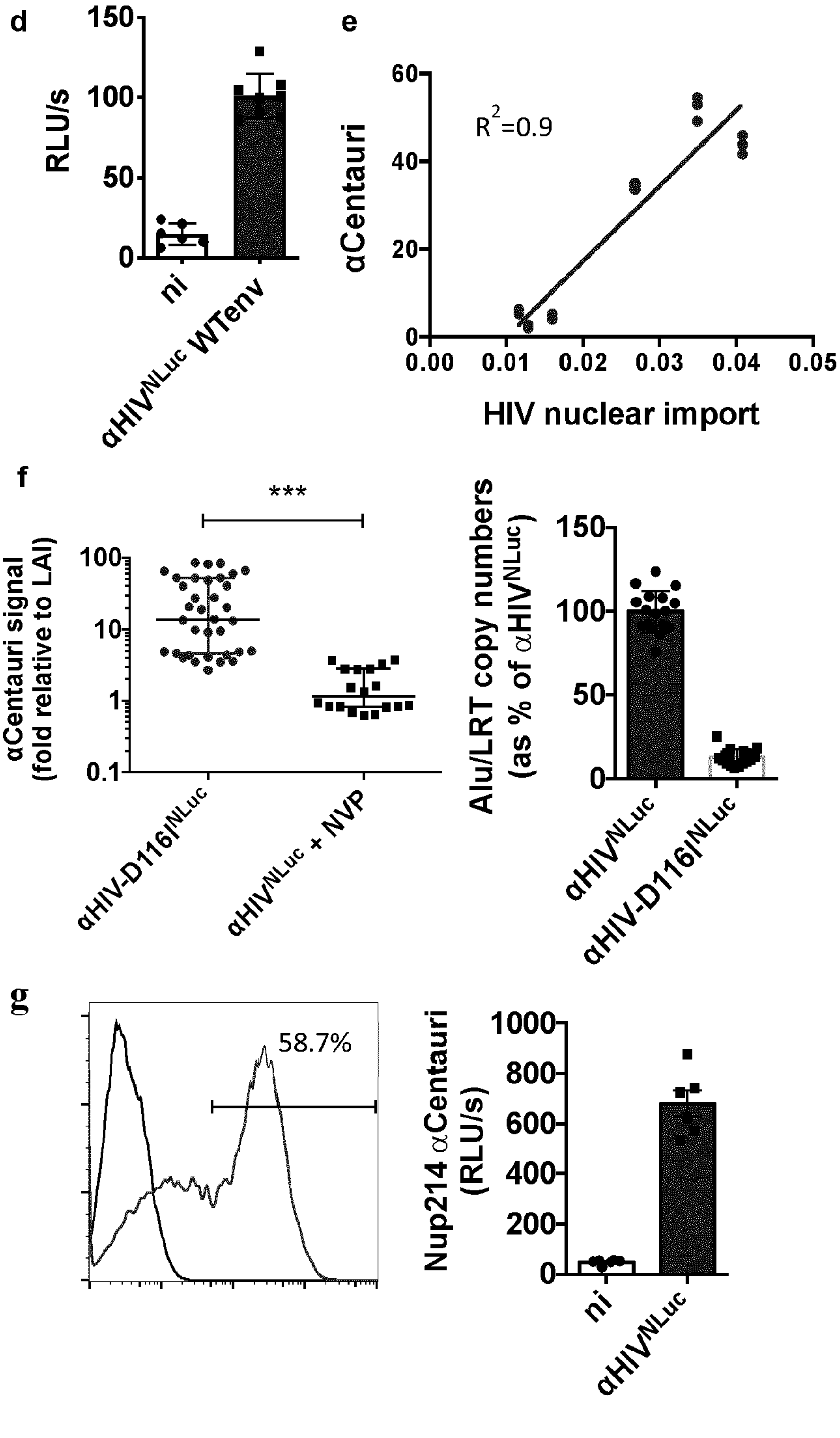


Figure 3 (final)

a

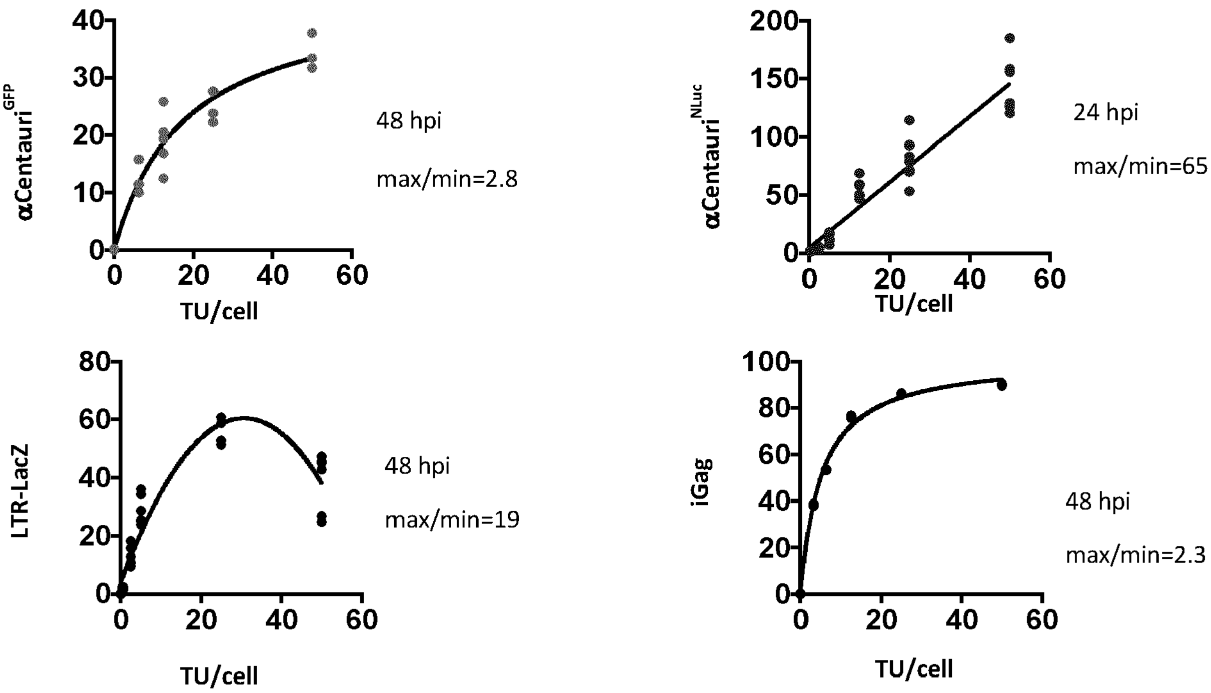


b

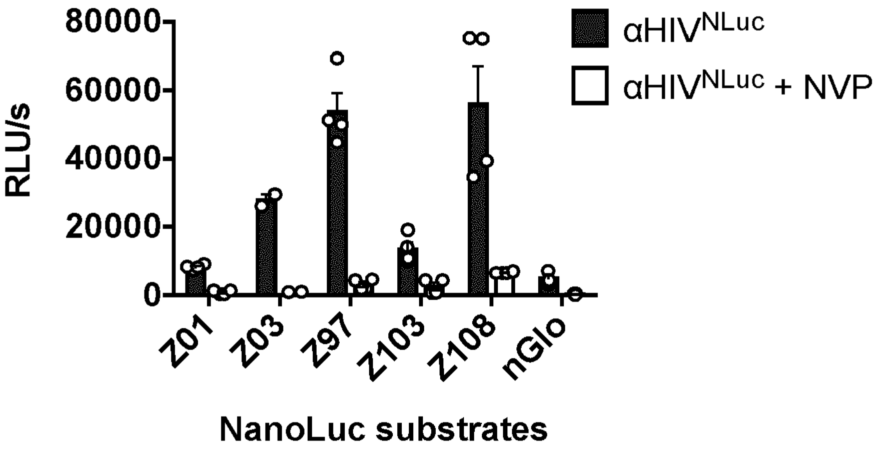


Figure 4 (continue)

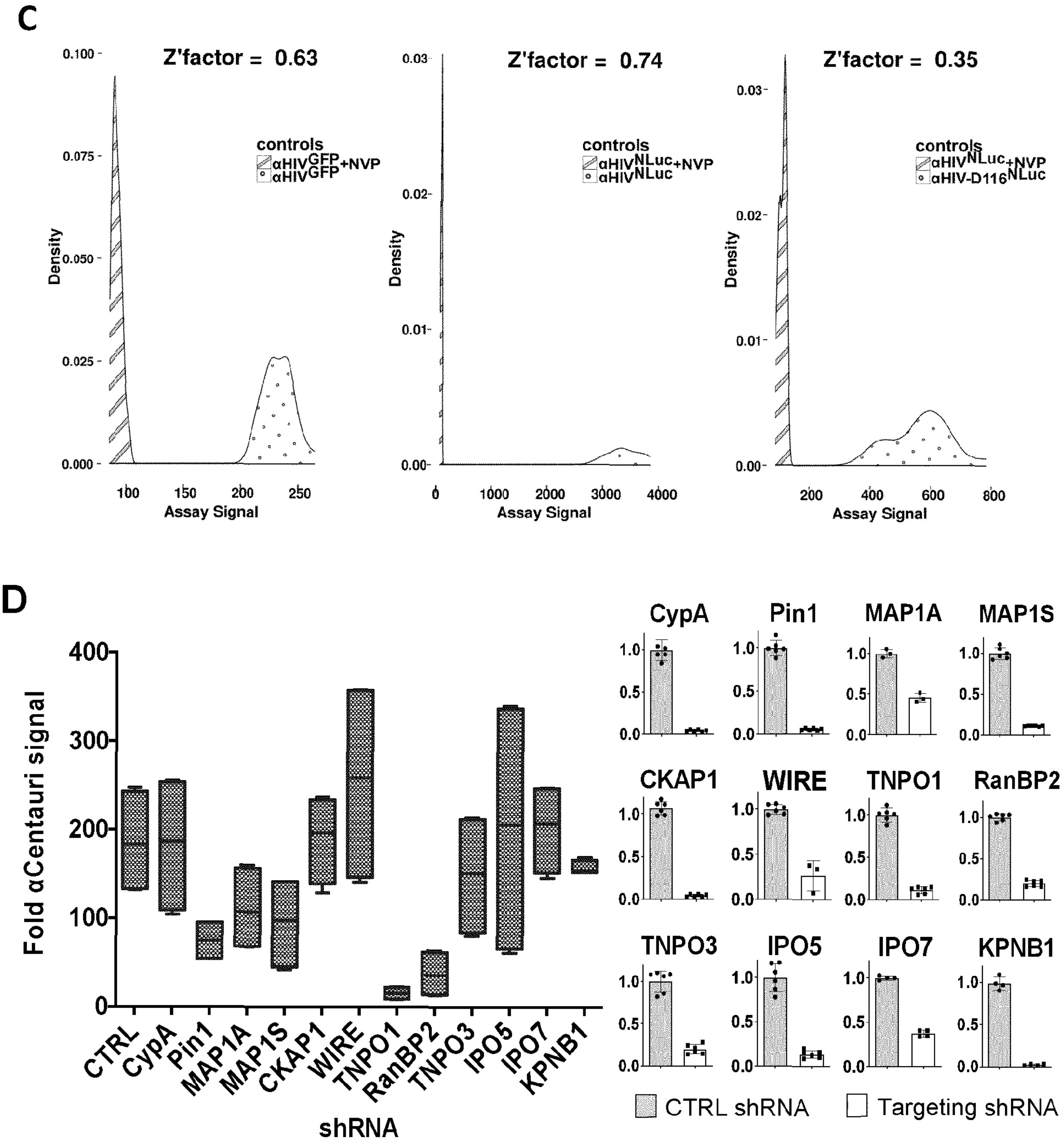


Figure 4 (Finale)

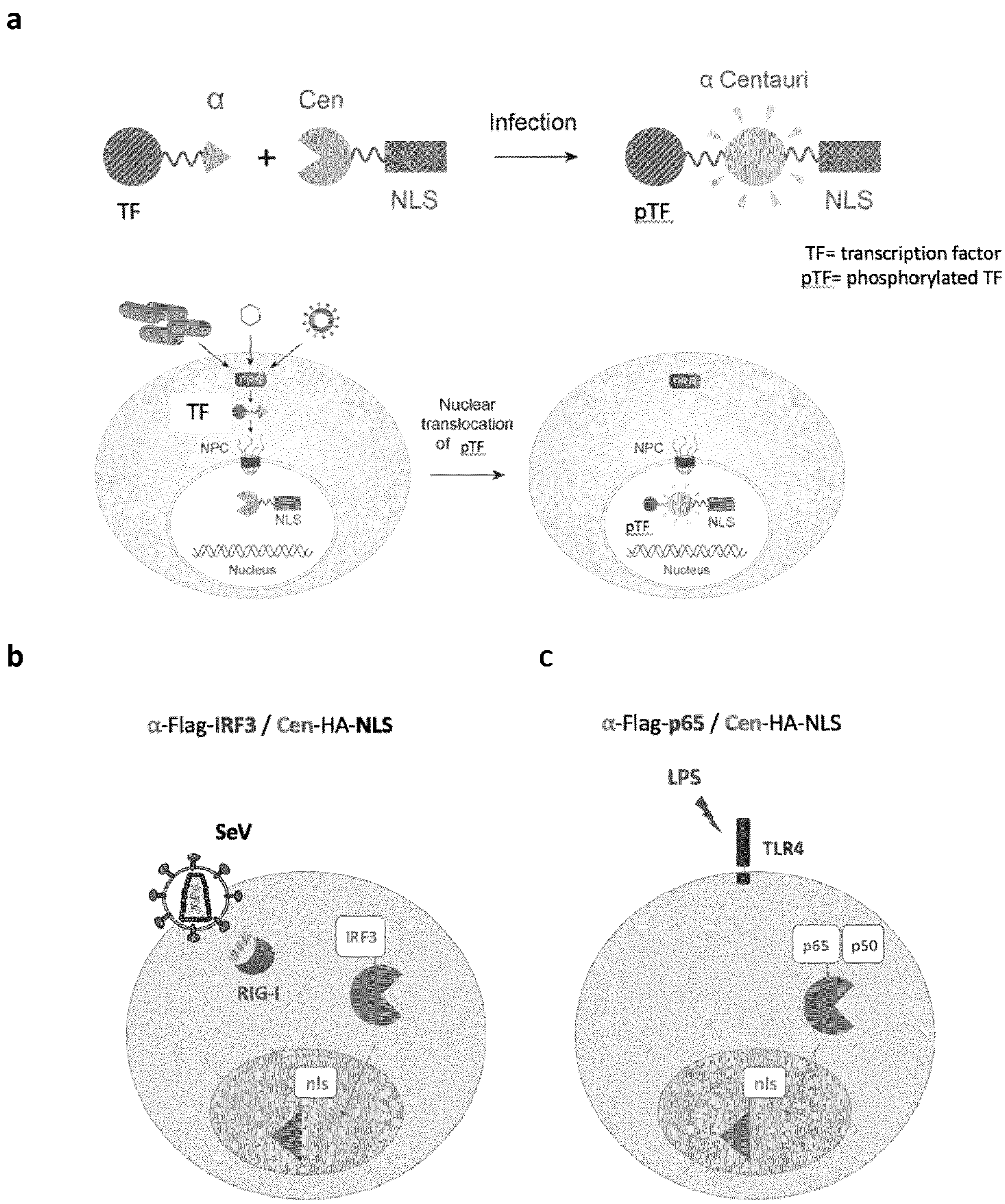
a
α
Cen
TF
NLS
Infection
α Centauri
pTF
NLS
TF= transcription factor
pTF= phosphorylated TF
PRR
TF
NPC
NLS
Nucleus
Nuclear translocation of pTF
PRR
NPC
pTF
NLS
Nucleus
b
c
α-Flag-IRF3 / Cen-HA-NLS
α-Flag-p65 / Cen-HA-NLS
SeV
RIG-I
IRF3
nls
LPS
TLR4
p65
p50
nls

Figure 5

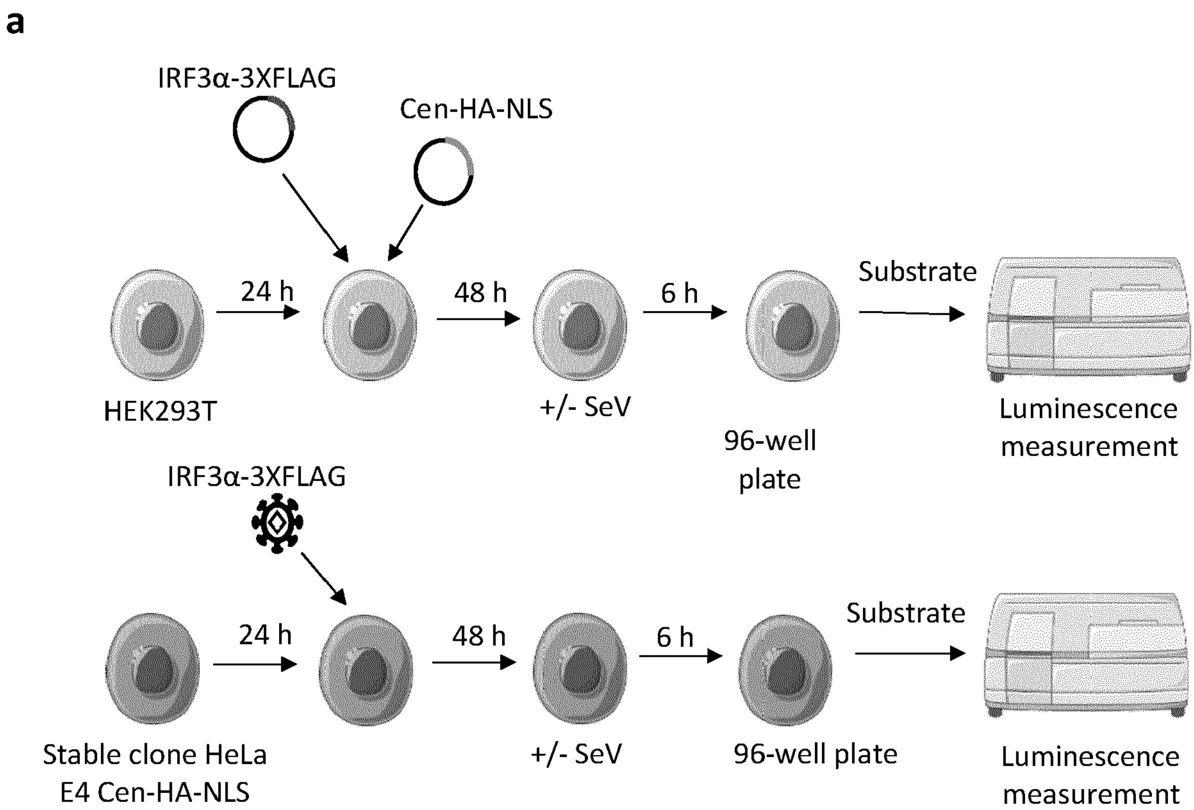


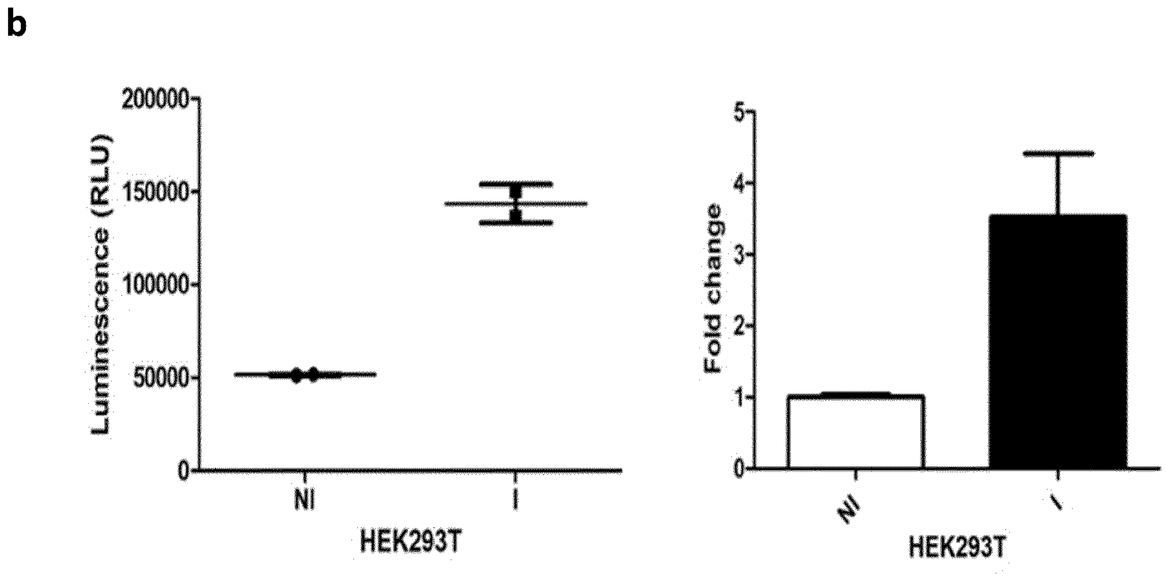


Figure 6 (continue)

c

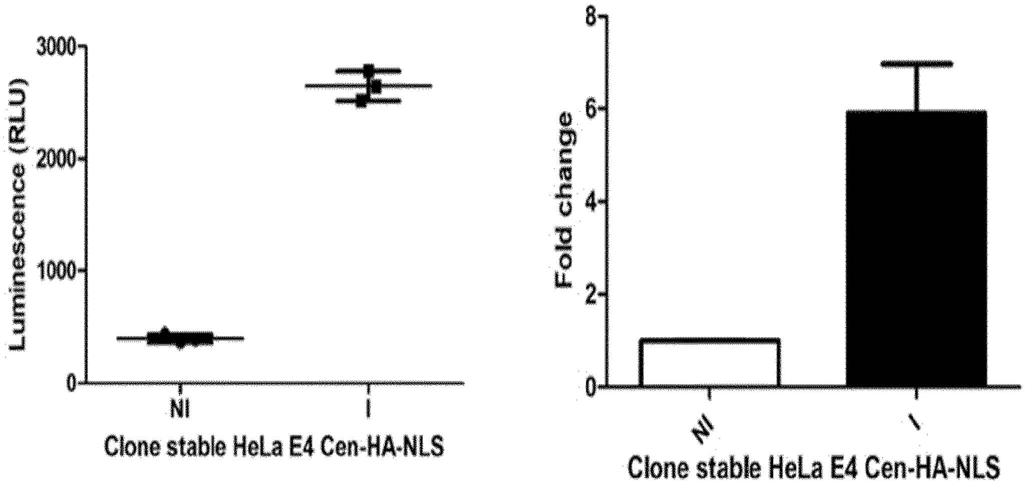


d

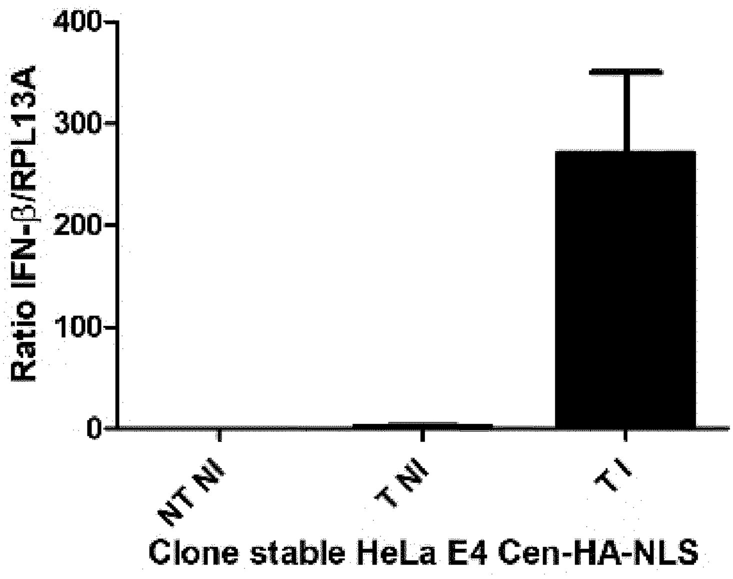


Figure 6 (Continue)

e

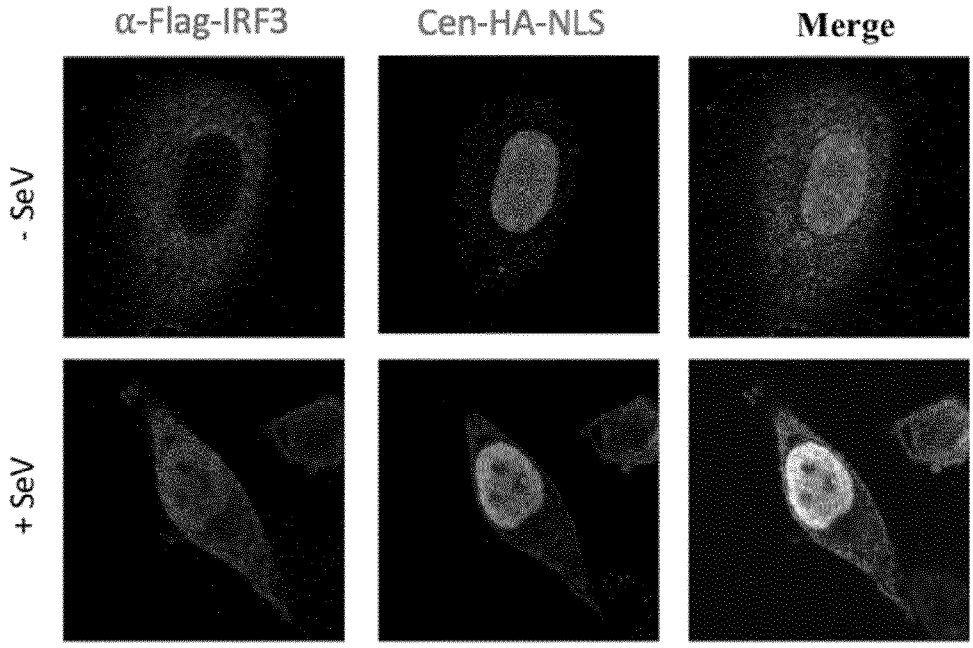


f

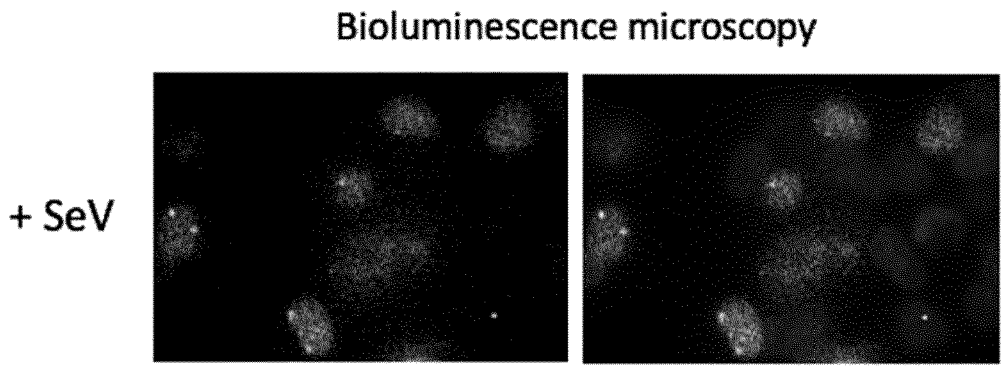


SeV=Sendai virus

Figure 6 (final)

a

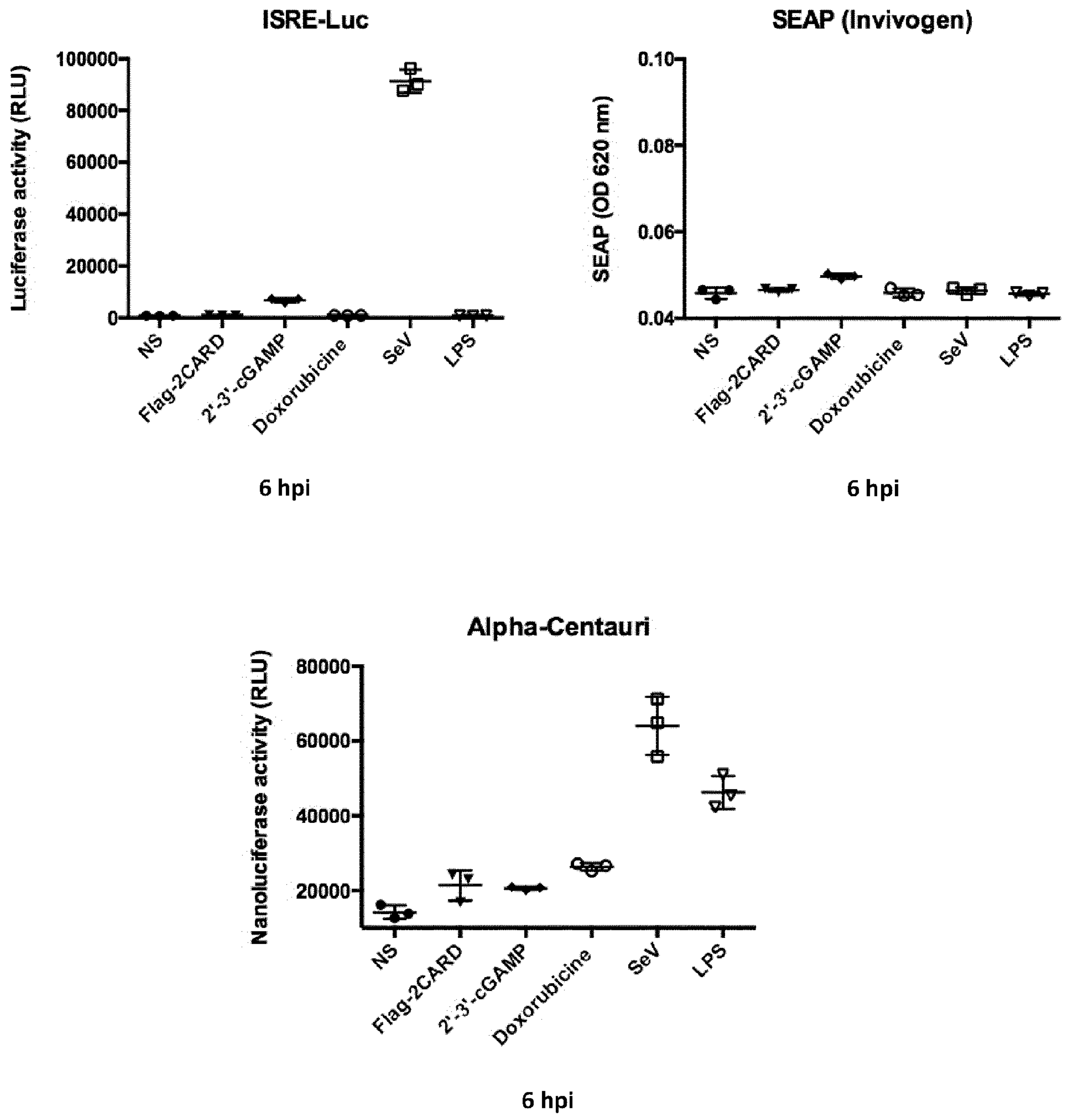


Figure 7 (Continue)

b

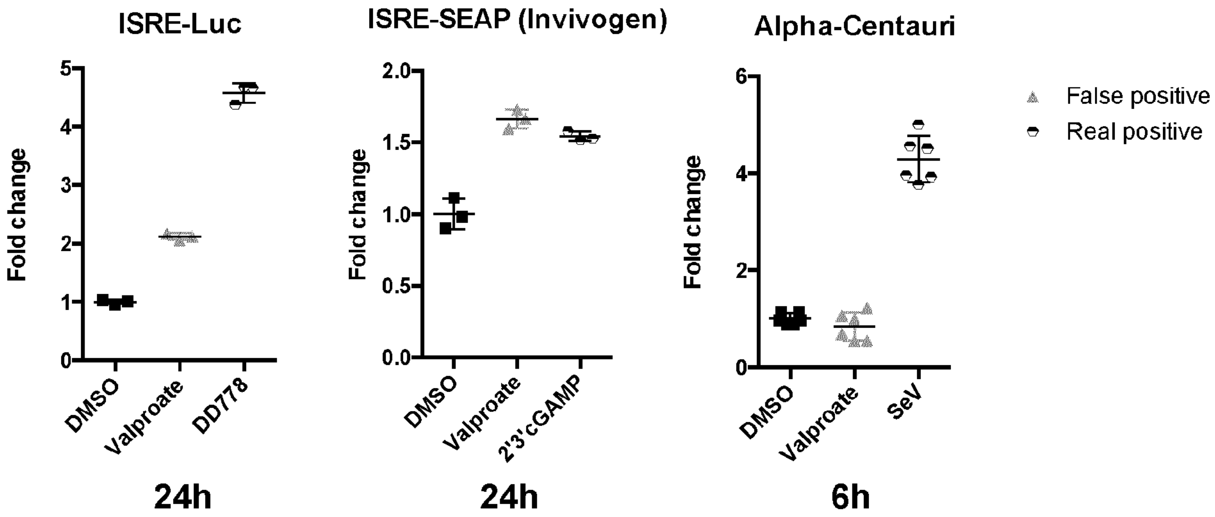


C

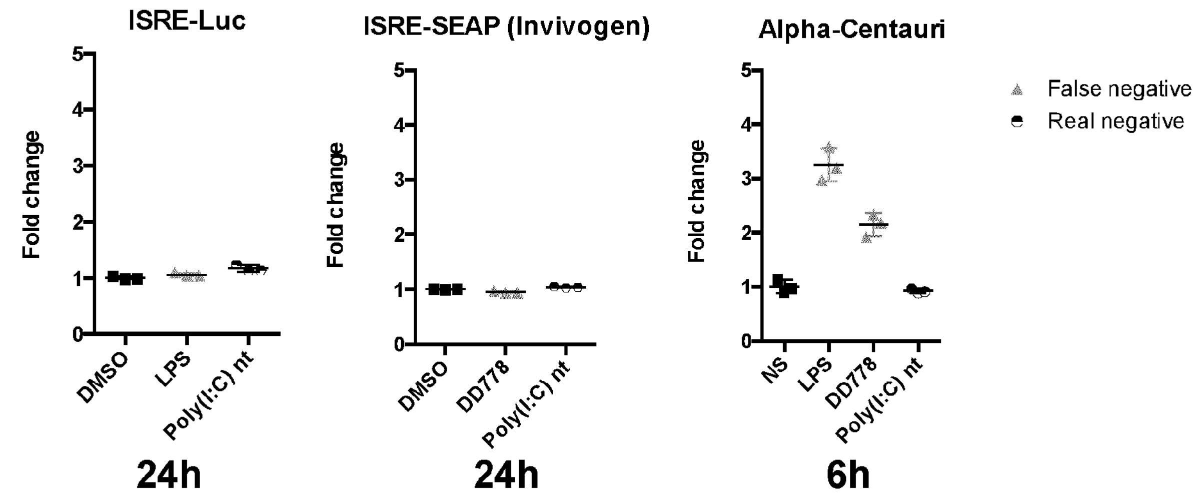


Figure 7 (Final)

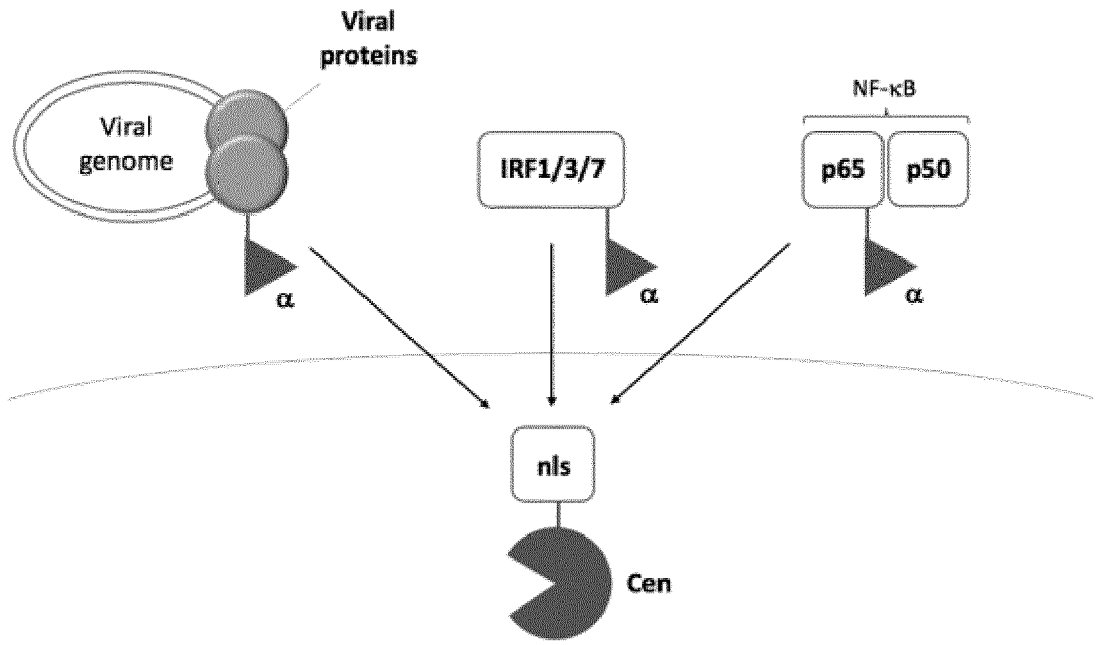


Figure 8

Europäisches Patentamt
European Patent Office
Office européen des brevets

# EUROPEAN SEARCH REPORT

Application Number
EP 20 30 6043

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/113838 A2 (UNIV LELAND STANFORD JUNIOR [US]; BLAU HELEN M [US] ET AL.) 1 December 2005 (2005-12-01)<br>* abstract *<br>* page 6, paragraph 22 *<br>* page 7, paragraph 26 *<br>* page 9, paragraph 35 *<br>* page 12, paragraphs 49,50 *<br>* page 15, paragraph 74 *<br>* page 23, paragraph 96 *<br>* page 38 - page 41; example 2 *<br>----- | 1-17 | INV.<br>C12N15/62<br>C12Q1/70 |
| X | WO 2006/062877 A2 (UNIV CALIFORNIA [US]) 15 June 2006 (2006-06-15)<br>* abstract *<br>* page 26, line 15 - page 27, line 19 *<br>* page 35, line 1 - line 24 *<br>* page 37, line 2 - line 25 *<br>* page 45, line 12 - line 27 *<br>----- | 1-17 | |
| X | WO 2004/070351 A2 (ODYSSEY THERA INC [US]) 19 August 2004 (2004-08-19)<br>* abstract *<br>* page 12, paragraph 5 *<br>* page 33, last paragraph - page 34, paragraph 2 *<br>* page 48 - page 50; example 5 *<br>* page 52 - page 55; examples 7,8 *<br>* page 71; claims 1,4 *<br>* page 77; claims 36,37 *<br>-----<br>-/-- | 1-17 | TECHNICAL FIELDS SEARCHED (IPC)<br>C12N<br>C12Q |

The present search report has been drawn up for all claims

1

| Place of search | Date of completion of the search | Examiner |
|---|---|---|
| Munich | 3 March 2021 | Grötzinger, Thilo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 30 6043

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/015161 A2 (UNIV ST LOUIS [US]) 17 February 2005 (2005-02-17) * abstract * * page 10, paragraph 35 * * page 11, paragraph 37 * * page 22, paragraph 90 * * page 39, paragraph 161 * * page 45, paragraph 178 - page 47, paragraph 183 * ----- | 1-17 | |
| | | | TECHNICAL FIELDS SEARCHED (IPC) |

1 The present search report has been drawn up for all claims

| Place of search | Date of completion of the search | Examiner |
|---|---|---|
| Munich | 3 March 2021 | Grötzinger, Thilo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 30 6043

This annex lists the patent family members relating to the patent documents cited in the above-mentioned European search report. The members are as contained in the European Patent Office EDP file on The European Patent Office is in no way liable for these particulars which are merely given for the purpose of information.

03-03-2021

| Patent document cited in search report | Publication date | Patent family member(s) | Publication date |
|---|---|---|---|
| WO 2005113838 A2 | 01-12-2005 | EP 1766095 A2 | 28-03-2007 |
| | | EP 2400030 A1 | 28-12-2011 |
| | | US 2005287522 A1 | 29-12-2005 |
| | | US 2012077204 A1 | 29-03-2012 |
| | | US 2014370522 A1 | 18-12-2014 |
| | | WO 2005113838 A2 | 01-12-2005 |
| WO 2006062877 A2 | 15-06-2006 | AU 2005314231 A1 | 15-06-2006 |
| | | CA 2638888 A1 | 15-06-2006 |
| | | EP 1828403 A2 | 05-09-2007 |
| | | US 2006257942 A1 | 16-11-2006 |
| | | WO 2006062877 A2 | 15-06-2006 |
| WO 2004070351 A2 | 19-08-2004 | AU 2004209398 A1 | 19-08-2004 |
| | | AU 2010224363 A1 | 14-10-2010 |
| | | CA 2514843 A1 | 19-08-2004 |
| | | EP 1590476 A2 | 02-11-2005 |
| | | JP 5122129 B2 | 16-01-2013 |
| | | JP 2006518853 A | 17-08-2006 |
| | | JP 2013015525 A | 24-01-2013 |
| | | US 2004161787 A1 | 19-08-2004 |
| | | US 2006224331 A1 | 05-10-2006 |
| | | US 2012149597 A1 | 14-06-2012 |
| | | WO 2004070351 A2 | 19-08-2004 |
| WO 2005015161 A2 | 17-02-2005 | US 2005144661 A1 | 30-06-2005 |
| | | WO 2005015161 A2 | 17-02-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BELOUSOV.** *Nucleic Acids Res.,* 1997, vol. 25, 3440-3444
- **AMARA et al.** *J.Virol.,* 2003
- **DIXON et al.** *ACS Chem. Biol.,* 2016
- **HALL, M.P. et al.** *ACS Chem Biol,* 2012, vol. 7, 1848-1857